# EUROPEAN PATENT APPLICATION

(11) **EP 4 442 825 A1**
(43) Date of publication of application: **09.10.2024**
(21) Application number: 22901386.7
(22) Date of filing: 30.11.2022
(51) Int. Cl.: C12N 15/11, C12N 15/113, C12N 15/115, C12Q 1/02, C12Q 1/68, C12Q 1/6897

(54) **RNA MOLECULE**

(30) Priority: 30.11.2021 JP 2021194197
(71) Applicant: Kyoto University, Kyoto-shi, Kyoto 606-8501 (JP)
(72) Inventor: SAITO, Hirohide, Kyoto-shi, Kyoto 606-8501 (JP); KAWASAKI, Shunsuke, Kyoto-shi, Kyoto 606-8501 (JP); KUWABARA, Takeru, Kyoto-shi, Kyoto 606-8501 (JP)
(74) Representative: HGF
(86) International application number: PCT/JP2022/044268
(87) International publication number: WO 2023/100955

(57) **Abstract**

An object of the present invention is to provide an RNA molecule that can be translationally activated by specifically responding to a wide range of target substances, and an RNA molecule that can specifically detect a target molecule without translation, and a translational regulation method, and a method for detecting a target substance using these RNA molecules. The present invention provides an RNA molecule comprising: (a) a recognition region that specifically recognizes a target substance; (b) a cap-independent translational activation region positioned on the 3' terminal side of the recognition region; and (c) a coding region positioned on the 3' terminal side of the translational activation region, the coding region being translated by the translational activation region, in which the RNA molecule does not contain a 5' cap structure, or an RNA molecule comprising: (A) a recognition region that specifically recognizes a target substance; and (B) a reporter region positioned on the 3' terminal side of the recognition region, in which the RNA molecule does not contain a 5' cap structure, and a translational regulation method and a method for detecting a target substance using these RNA molecules.

## Description

### TECHNICAL FIELD

The present invention relates to an RNA molecule that can be translationally activated by specifically responding to a target substance, and an RNA molecule that can specifically detect a target molecule.

### BACKGROUND ART

As many costly medications such as molecular targeting drugs or cell-based therapy have recently come into use, there has been a strong demand for efficient medical interventions based on individualized treatments. Such a demand can be met by an artificial regulation technique using transcription of DNA or translation of RNA, which caused increased interest only in particular cells or organs. In particular, translational regulation using RNA has received attention because it exerts less effect on the human genome and is safer than transcriptional regulation methods.

Under these circumstances, an attempt to construct an artificial translational regulation circuit has been a major area of interest in the field of genetic engineering in recent years. Many studies have been made, for example, on the development of translational regulation devices that may be used in such a circuit, the expansion of the type of a substance of interest to which the device responds, or specific circuit design by the combination of devices (see, for example, Non-Patent Literature 1 to 5).

Biosensors have been developed to which techniques of constructing such biomolecular devices and circuits based on RNA are used without the need to introduce RNA into cells. There are reports, for example, of successful detection of Ebola virus RNA (see, for example, Non-Patent Literature 6) and detection of various contaminants in water (see, for example, Non-Patent Literature 7). Thus, this technique is one of the advanced technologies that have drawn most attention in recent years, and it is very practical.

The type of a substance of interest to which a translational regulation device is capable of responding depends largely on the design and driving principle of the device. Various attempts have been made to expand the type of the substance of interest to which the device is capable of responding. Various systems exist, such as a system designed to detect freely selected RNA (e.g., Non-Patent Literature 1 and 2) and a system designed in order to detect an RNA-binding protein through the use of the binding of the protein to a specific RNA sequence (e.g., Non-Patent Literature 3 and 4).

### REFERENCE DOCUMENT LIST

### NON-PATENT LITERATURE

Non-Patent Document 1: Green, et al., Cell 159, 925-939 (2014)
Non-Patent Document 2: Miki, et al., Cell Stem Cell 16, 1-13 (2015)
Non-Patent Document 3: Auslander, et al., Nat Methods 11, 1154-1160 (2014).
Non-Patent Document 4: DiAndreth, et al., bioRxiv. doi.org/10.1101/2019.12.15.867150
Non-Patent Document 5: Mustafina, et al., ACS Synth. Biol. 9, 1, 19-25 (2020)
Non-Patent Document 6: Pardee, et al., Cell 159, 940-954 (2014)
Non-Patent Document 7: Jung, et al., Nat Biotechnol 38, 1451-1459 (2020).

### SUMMARY OF INVENTION

### PROBLEM TO BE SOLVED BY THE INVENTION

However, it has been difficult to develop a module having a unitary structure that can detect many types of intracellular molecules such as RNAs, proteins, and intracellular small molecules. Furthermore, no system that can activate gene expression by introducing *in vitro* transcribed mRNA to cells and responding to a wide range of molecules has yet been developed.

The present inventors have earnestly researched and, as a result, completed the present invention by finding that ON switch RNA that is translationally activated by specifically responding to a wide range of target substances from biomolecules such as proteins or RNAs to low-molecular compounds, and ON switch RNA that generates a signal through a method other than translation by specifically responding to a target substance, can be designed by using an RNA molecule based on an mRNA structure without a 5' cap structure, and this uses a single operating principle.

Specifically, the present invention includes the following aspects.
[1] An RNA molecule including:
   (a) a recognition region that specifically recognizes a target substance;
   (b) a cap-independent translational activation region positioned on the 3' terminal side of the recognition region; and
   (c) a coding region positioned on the 3' terminal side of the translational activation region, the coding region being translated by the translational activation region, in which
      the RNA molecule does not contain a 5' cap structure.
[2] The RNA molecule according to [1], in which the recognition region includes a nucleic acid sequence selected from an RNA aptamer, aptazyme, and a nucleic acid sequence that specifically recognizes RNA.
[3] The RNA molecule according to [1] or [2], in which the translational activation region is selected from an internal ribosome entry site (IRES), a ribosome binding site, and a translational activator recognition site.
[4] The RNA molecule according to any one of [1] to [3], in which the coding region includes a nucleic acid sequence encoding a detectable protein, a therapeutic protein, or an RNA-binding protein.
[5] The RNA molecule according to any one of [1] to [4], further comprising a spacer region linked to the 5' side of the recognition region.
[6] The RNA molecule according to any one of [1] to [5], in which
   the recognition region is linked to the 5' side of the translational activation region, and
   the translational activation region is linked to the 5' side of the coding region.
[7] An RNA molecule comprising:
   (A) a recognition region that specifically recognizes a target substance; and
   (B) a reporter region positioned on the 3' terminal side of the recognition region, in which
      the RNA molecule does not contain a 5' cap structure.
[8] The RNA molecule according to [7], in which the recognition region includes a nucleic acid sequence selected from an RNA aptamer, aptazyme, and a nucleic acid sequence that specifically recognizes RNA.
[9] The RNA molecule according to [7] or [8], in which the reporter region includes a light-up aptamer that generates an optical signal by binding to an RNA probe.
[10] A DNA construct comprising:
   a DNA sequence encoding an RNA molecule according to any one of [1] to [9]; and
   a 5' cap structure-removing sequence.
[11] The DNA construct according to [10], in which the 5' cap structure-removing sequence encodes self-cleaving ribozyme, a miRNA target sequence, or an RNA-degrading sequence.
[12] A method for producing an RNA molecule according to any one of [1] to [9], including steps of:
   obtaining an RNA molecule comprising: a 5' cap structure; a recognition region that specifically recognizes a target substance; a cap-independent translational activation region positioned on the 3' terminal side of the recognition region; and a coding region positioned on the 3' terminal side of the translational activation region, the coding region being translated by the translational activation region; and
   removing the 5' cap structure from the RNA molecule.
[13] A method for detecting a target substance, comprising the step of bringing an RNA molecule according to any one of [1] to [9] into contact with a cell. the RNA molecule according to claim 1 or 7 into contact with a cell
[14] A method for detecting a target substance, including introducing a DNA construct according to [10] or [11] to a cell.
[15] A method for detecting a target substance, including adding exoribonuclease and an RNA molecule according to any one of [1] to [6] or a DNA construct comprising a DNA sequence encoding the RNA molecule and a 5' cap structure-removing sequence to a cell-free gene expression system.
[16] A method for detecting a target substance, including steps of:
   adding exoribonuclease, an RNA molecule according to any one of [7] to [9] or a DNA construct comprising a DNA sequence encoding the RNA molecule and a 5' cap structure-removing sequence, and an RNA probe that generates an optical signal by binding to the reporter region of the RNA molecule, to a sample; and
   detecting the optical signal.
[17] A translational regulation system including an RNA molecule according to any one of [1] to [6] or a DNA construct including a DNA sequence encoding the RNA molecule and a 5' cap structure-removing sequence.
[18] A translational regulation system including at least two or more groups of RNA molecules, in which
   each of the groups of RNA molecules includes a plurality of RNA molecules each comprising: a recognition region that specifically recognizes a target substance; a cap-independent translational activation region positioned on the 3' terminal side of the recognition region; and a coding region positioned on the 3' terminal side of the translational activation region, the coding region being translated by the translational activation region, in which each of the RNA molecules does not contain a 5' cap structure, and
   in which the target substance that is specifically recognized by the recognition region differs for each of the group of RNA molecules.

### EFFECTS OF INVENTION

The present invention employs the driving principle involving the stabilization of an RNA molecule by a target substance, followed by translational promotion from a sequence subsequent to IRES, or signal generation through complex formation between an RNA molecule and a substrate without translation. The target substance that is used for the stabilization of an RNA molecule is not limited by its type such as RNA, a protein, or an intracellular small molecule. Therefore, a module having a unitary configuration capable of responding to target substances consisting of diverse molecules has been successfully developed. This configuration of the module may achieve preparation of a device for detecting any substance of interest that specifically interacts with RNA can be confirmed. This can be applied to detect an intracellular molecule and cause a response of interest such as cell death, on the basis of the same design principle. Alternatively, a target substance can be detected, without limitations to an intracellular manner, by detecting the generation of a signal in a desired sample solution. The RNA molecule according to the present invention further exerts the following effects.

### Development of general-purpose platform

Conventionally developed artificial translational regulation modules differ in design and vary in the types of detectable substances of interest (target substances). Hence, in order to detect a particular intracellular molecule, a translational regulation device designed according to the type of the molecule must be used. The present invention can employ the RNA molecule according to the present invention as a module having a unitary configuration based on a simple driving principle, irrespective of the type of the molecule of a target substance. Thus, it is not necessary to design a device from scratch even if the type of target substance is broadened. Since a very wide variety of target molecules can be used as triggers that induce the response of an RNA molecule, a large-scale translational regulation circuit can also be constructed using many RNA molecules.

### Development of translational activation (ON switch) system capable of introducing RNA

Most of the conventionally developed artificial translational regulation modules are OFF switch systems that repress translation in response to the presence of an intracellular molecule. Although some ON switch systems exist, problems have arisen such as difficulty in driving the systems by the introduction of *in vitro* transcribed mRNA in principle, and limitations on a target substance. The system of the present invention can be driven even by direct introduction of an RNA molecule, and its driving can also be achieved by using a wide range of molecules as target substances.

Due to these features, an RNA molecule according to the present invention can be used in cell engineering and clinical application *in vitro,* and in addition, can be used, without limitation intracellularly, in a cell-free gene expression system or as a biosensor for detecting a target substance under conditions that permit enzymatic reaction.

### BRIEF DESCRIPTION OF DRAWINGS

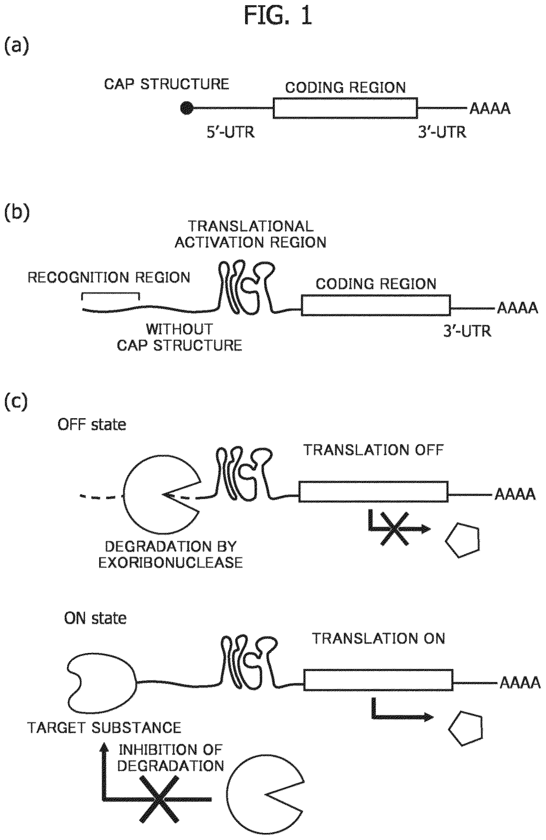

FIG. 1 is a diagram illustrating the design of the RNA molecule according to the present invention. FIG. 1(a) schematically shows typical mRNA having a cap structure at the 5' end. FIG. 1(b) shows the RNA molecule according to the present invention having no cap structure in 5'-UTR and having a recognition region and a translational activation region. FIG. 1(c) is a diagram illustrating an RNA molecule according to the present invention that is degraded by intracellular exoribonuclease in the absence of a target substance (OFF state) while the degradation of the ON switch is suppressed in the presence of a target substance (ON state) through the specific binding of the recognition region embedded in the RNA molecule to the target substance so that a freely selected protein encoded by a coding region is translated.

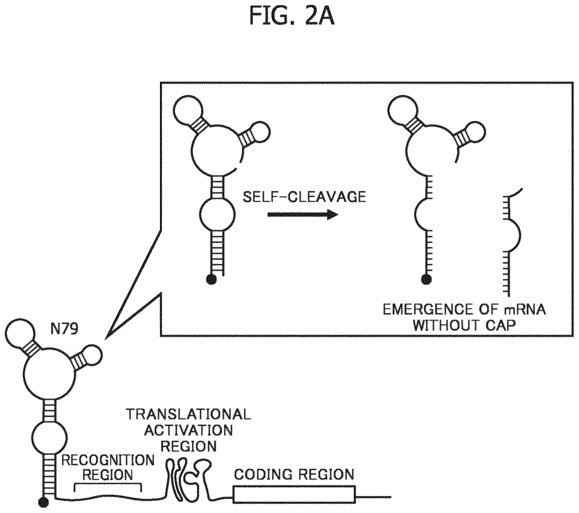

FIG. 2A is a diagram illustrating the design of a plasmid for intracellular expression of mRNA. In the diagram, an aptamer capable of recognizing a target substance was inserted to the 3' side of N79, a type of self-cleaving ribozyme, and then, IRES and a freely selected open reading frame (ORF) were placed to construct a plasmid-based mRNA ON switch.

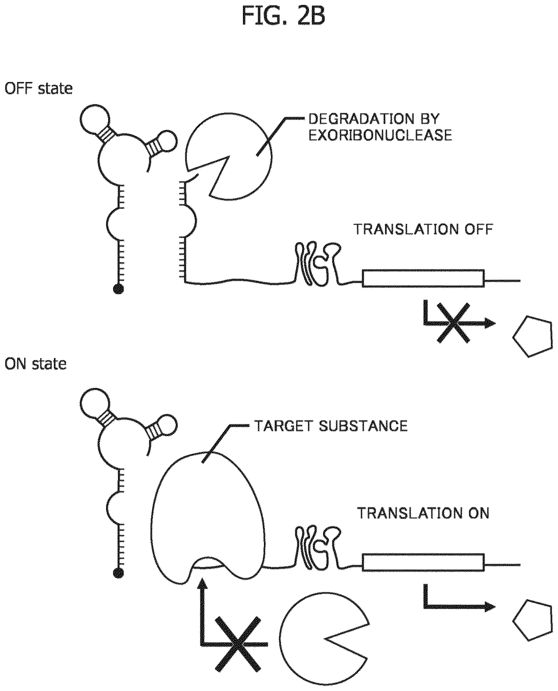

FIG. 2B is a diagram schematically illustrating the function of a mRNA ON switch. In the absence of Cas protein used as an exemplary target protein, this mRNA is intracellularly degraded because it lacks a cap structure, and as a result, its translation is repressed (OFF state, upper). By introducing the Cas protein, the degradation of the switch by exoribonuclease is presumably suppressed because the Cas protein specifically recognizes and binds to crRNA/sgRNA embedded in the mRNA (ON state, lower).

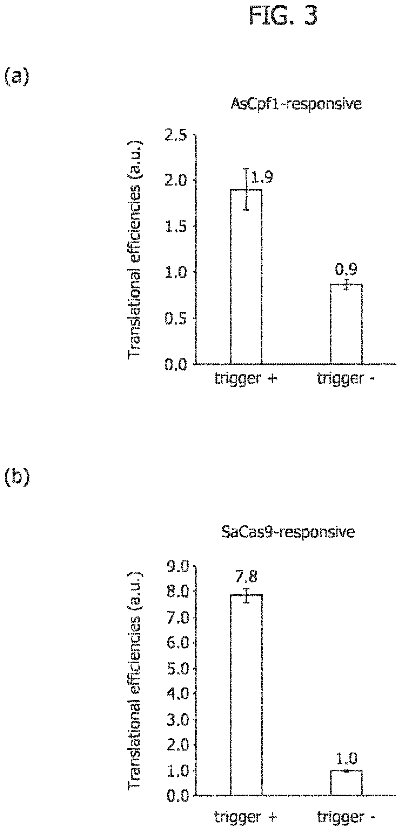

FIG. 3(a) is a graph showing the translation efficiency of a plasmid for the expression of an ON switch encoding a fluorescent protein EGFP with AsCpfl as a target substance. FIG. 3(b) is a graph showing the translation efficiency of a plasmid for the expression of an ON switch encoding a fluorescent protein with SaCas9 as a target substance.

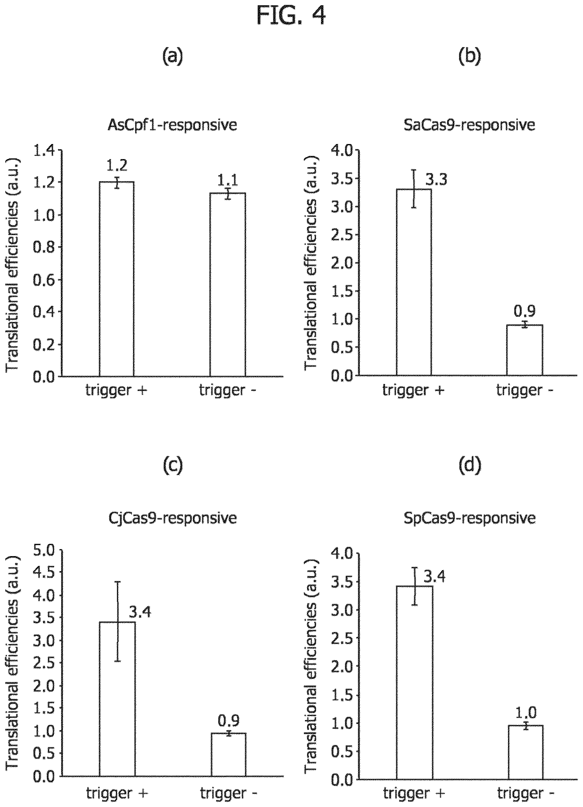

FIG. 4 is a graph showing the translation efficiency of *in vitro* transcribed ON switch mRNA encoding a fluorescent protein EGFP with AsCpf1 (a), SaCas9 (b), CjCas9 (c), or SpCas9 (d) as a target substance.

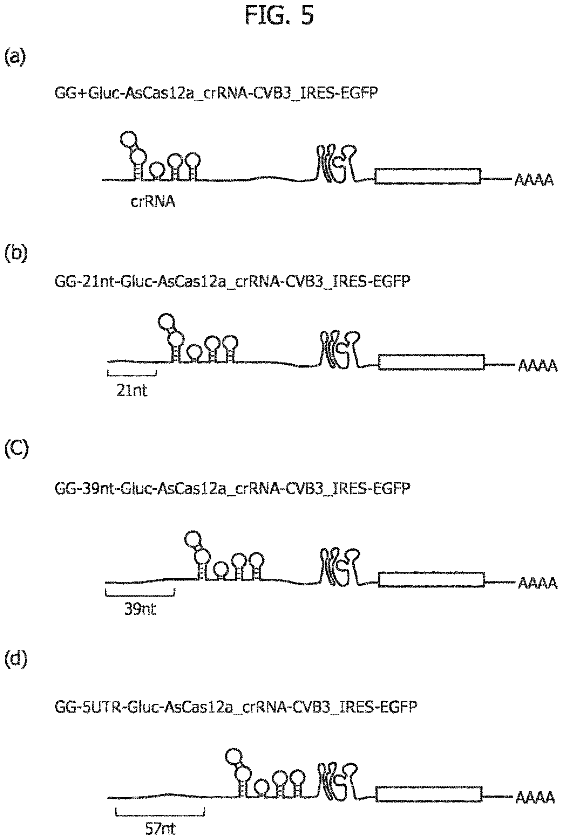

FIG. 5 is the design of an RNA molecule having a spacer region on the 5' side of a recognition region. FIG. 5(a) is a diagram schematically showing GG+Gluc-AsCas12a_crRNA-CVB3_IRES-EGFP (used in FIG. 4(a)). FIG. 5(b) is a diagram schematically showing GG-21nt-Gluc-AsCas12a_crRNA-CVB3_IRES-EGFP provided with a 21-nt spacer. FIG. 5(c) is a diagram schematically showing GG-39nt-Gluc-AsCas12a_crRNA-CVB3_IRES-EGFP provided with a 39-nt spacer. FIG. 5(d) is a diagram schematically showing GG-5'UTR-Gluc-AsCas12a_crRNA-CVB3_IRES-EGFP provided with a 57-nt spacer.

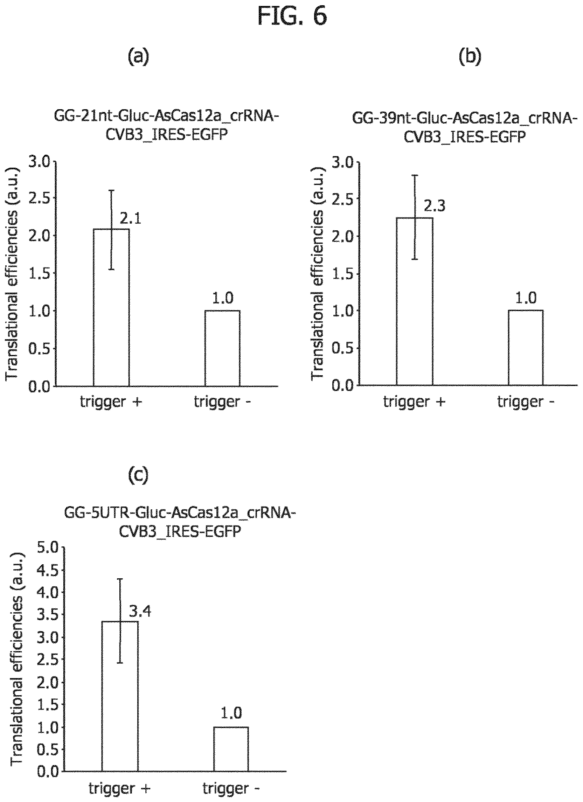

FIG. 6 is a graph showing the translation efficiency of each ON switch mRNA of GG-21nt-Gluc-AsCas12a_crRNA-CVB3_IRES-EGFP (a), GG-39nt-Gluc-AsCas12a_crRNA-CVB3IRES-EGFP (b), or GG-5'UTR-Gluc-AsCas12a_crRNA-CVB3_IRES-EGFP (c).

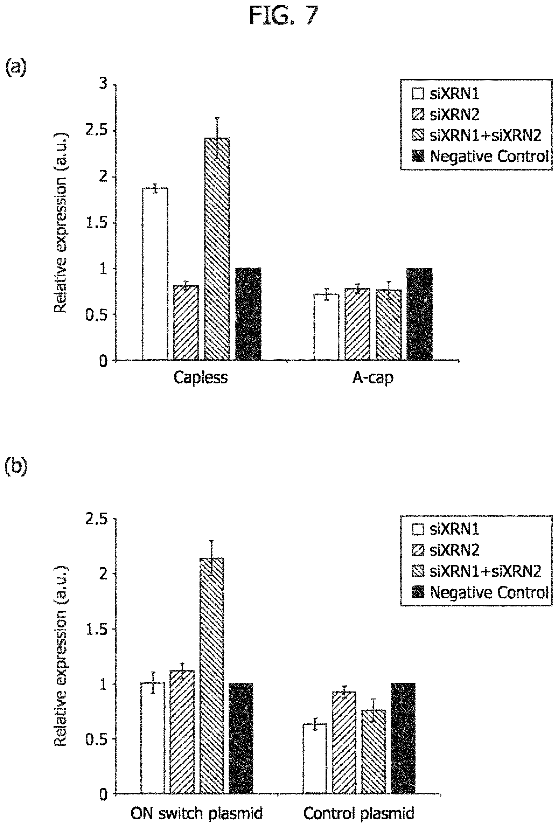

FIG. 7(a) is a graph showing translation efficiency when a mRNA ON switch synthesized by *in vitro* transcription was introduced to XRN-knockdown cells. FIG. 7(b) is a graph showing translation efficiency when a plasmid-based mRNA ON switch was introduced thereto.

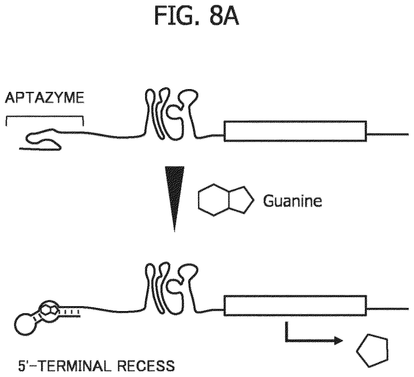

FIG. 8A is the design of an RNA molecule with an exemplary small molecule guanine as a target substance, and is a diagram schematically showing an RNA molecule having a guanine-responsive aptazyme in a recognition region (upper), and an RNA molecule that assumes a 5'-terminally recessed structure by adding guanine thereto (lower).

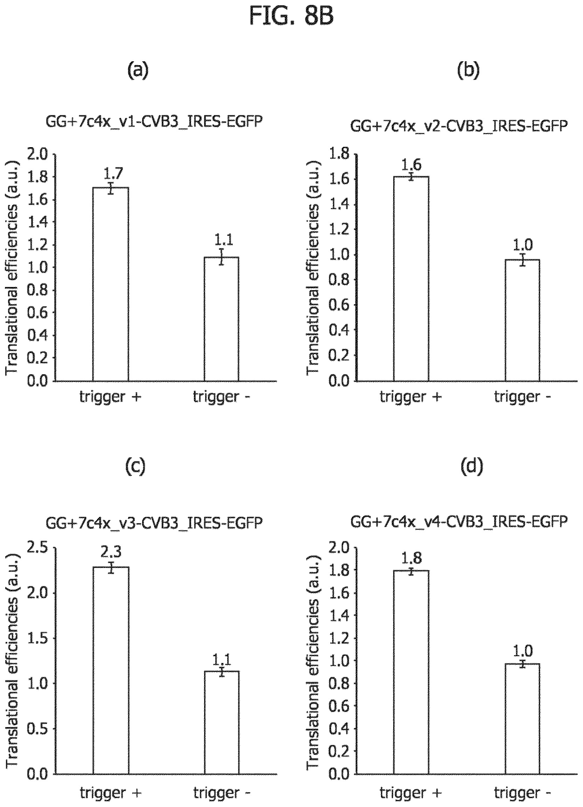

FIG. 8B is a graph showing the translation efficiency of each ON switch mRNA of GG+7c4x_v1-CVB3_IRES-EGFP (a), GG+7c4x_v2-CVB3_IRES-EGFP (b), GG+7c4x_v3-CVB3_IRES-EGFP (c), or GG+7c4x_v4-CVB3_IRES-EGFP (d).

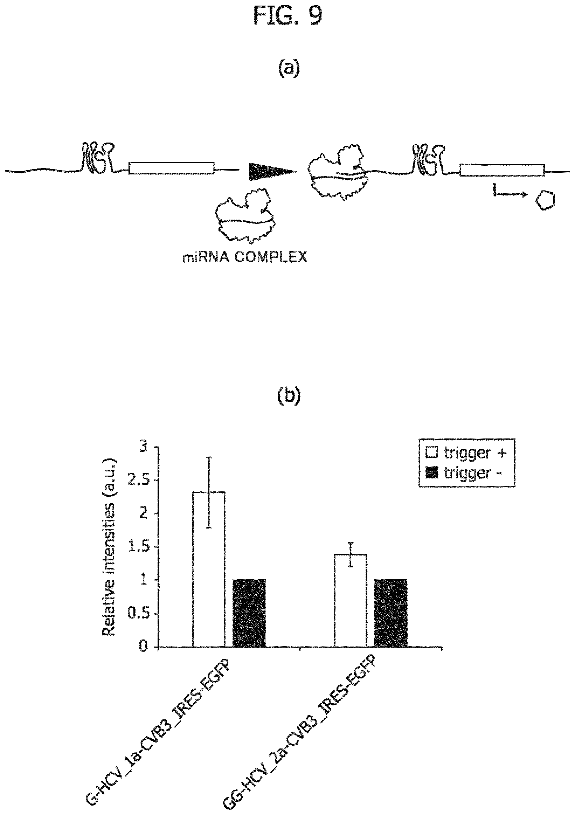

FIG. 9(a) is the design of an RNA molecule with a miR-122/RISC complex as a target substance. FIG. 9(b) is a graph showing the translation efficiency of G-HCV_1a-CVB3IRES-EGFP ON switch mRNA having the sequence of HCV RNA of genotype 1a, and G-HCV_2a-CVB3_IRES-EGFP ON switch mRNA having the sequence of HCV RNA of genotype 2a.

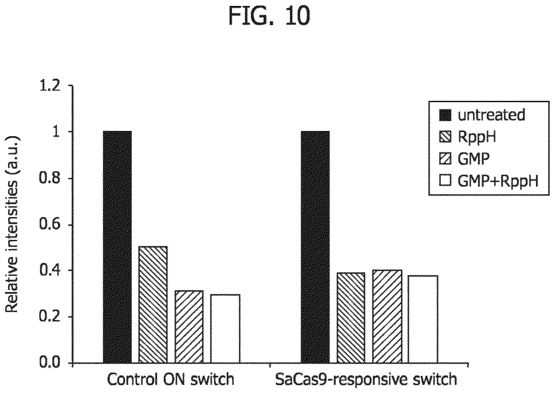

FIG. 10 shows results of studying leaky expression in the absence of a target substance (OFF state) depending on difference in the 5'-terminal structure of an RNA molecule. As for each of a control ON switch having no recognition region for a target substance (left) and an ON switch with SaCas9 as a target substance (right), a graph shows fluorescence intensities in the OFF state as "untreated" in which an mRNA ON switch prepared by *in vitro* transcription did not undergo additional treatment, "RppH" in which after transcription, the 5' end was subjected to dephosphorylation treatment with RppH, "GMP" in which at the time of transcription, the 5' end was monophosphorylated by using GMP, and "GMP+RppH" in which at the time of transcription, GMP was used, and after transcription, the 5' end was subjected to dephosphorylation treatment with RppH.

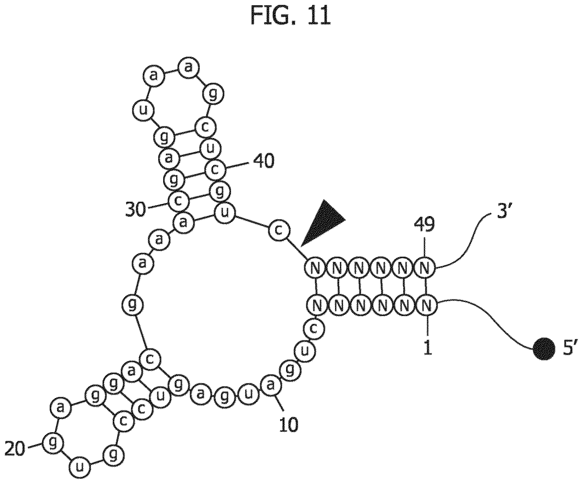

FIG. 11 is a structural prediction diagram of hammerhead ribozyme (HHR) which is a modified product of tobacco ringspot virus-derived ribozyme. The arrow shows a self-cleavage site, and the RNA sequence of HHR is shown in SEQ ID NO: 49.

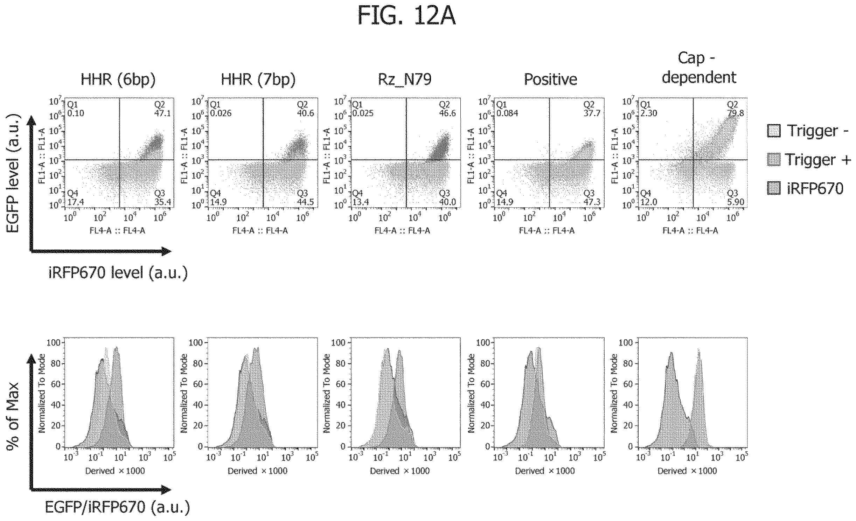

FIG. 12A is a diagram showing, in dot plots and histograms, change in cell population when each construct of a SaCas9-responsive mRNA switch containing HHR (6 bp), a SaCas9-responsive mRNA switch containing HHR (7 bp), a SaCas9-responsive switch using N79, a reporter plasmid having no cap-removing mechanism for the expression of EGFP, or a reporter plasmid for the expression of EGFP via a 5' cap was introduced to the cell population.

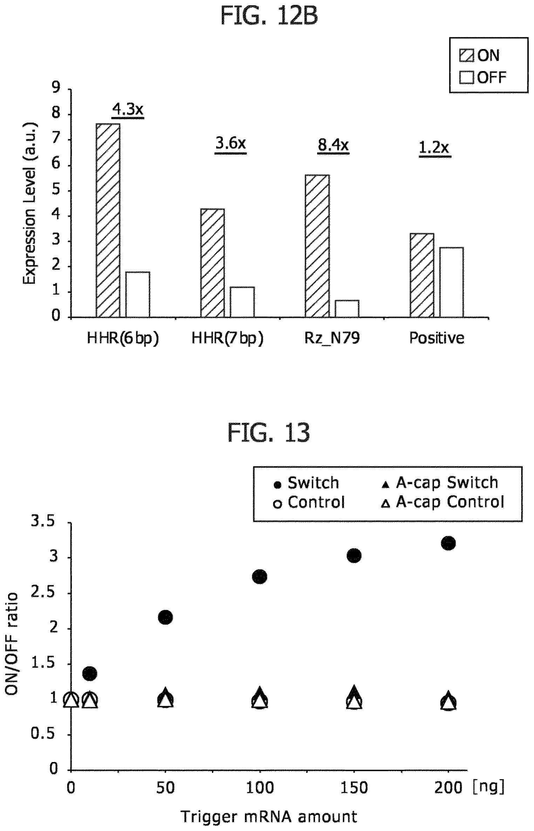

FIG. 12B is a graph showing a median value of EGFP/iRFP670 brightness in each construct.
FIG. 13 is a graph showing the translational activation efficiency of each trigger mRNA when an ON/OFF reporter expression level without trigger mRNA introduction is defined as 1.

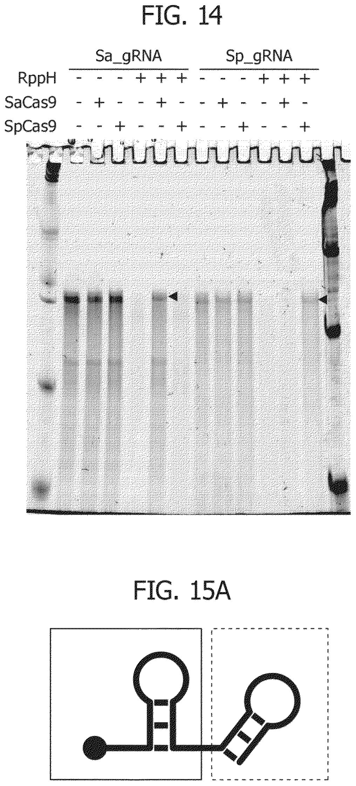

FIG. 14 shows gel electrophoresis results after reaction under each condition of SaCas_gRNA or SpCas_gRNA. As shown in the figure, the degradation of each RNA was inhibited only in the absence of RppH or when binding Cas9 protein was added (arrow) and occurred in the presence of RppH (XRN1 activity).
FIG. 15A is a conceptual diagram showing the design of a complex of a fluorescent RNA aptamer (RNA probe) which is an exemplary RNA molecule according to the second embodiment of the present invention. The solid line box represents a ligand-binding site corresponding to a recognition region, and the broken line box represents a fluorescent aptamer site corresponding to a reporter region.

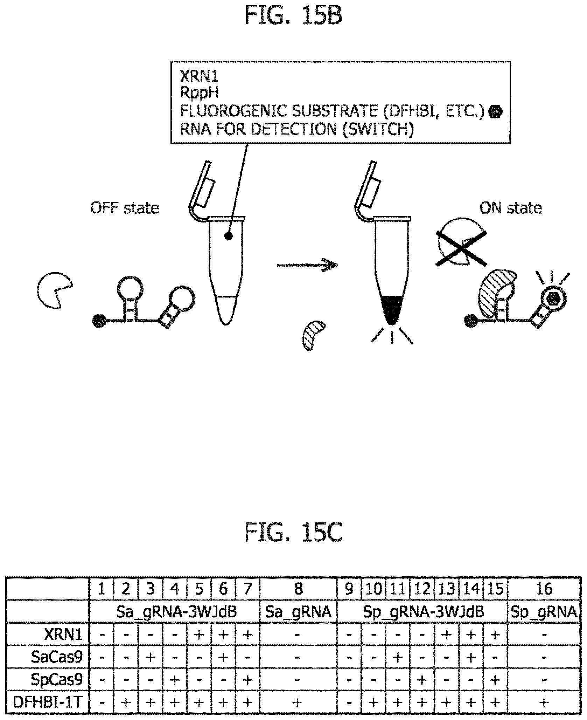

FIG. 15B is a diagram showing an exemplary detection mechanism of a target substance by a fluorescent RNA aptamer. A reaction system contains XRN1, RppH, a fluorogenic substrate, and a fluorescent RNA aptamer (the RNA molecule according to the second embodiment). When a target substance (Cas protein) is absent in the reaction system, complexed RNA is degraded by XRN1 and therefore emits no fluorescence (OFF state). In the presence of a target substance, the degradation of complexed RNA is inhibited so that fluorescence is generated by the action of the fluorescent aptamer and the fluorogenic substrate (ON state).
FIG. 15C shows a condition of the presence or absence (+/-) of a substance and an enzyme contained in each sample reaction system, and the type of sgRNA.

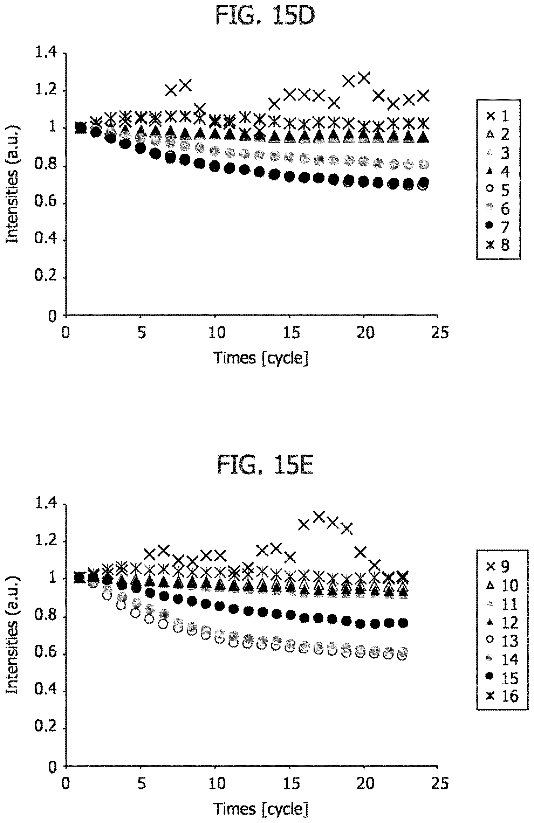

FIG. 15D shows change in fluorescence under each condition of SaCas9-binding RNA.
FIG. 15E shows change in fluorescence under each condition of SpCas9-binding RNA.

### MODE FOR CARRYING OUT THE INVENTION

Hereinafter, embodiments of the present invention will be described. However, the present invention is not limited by the embodiments described below.

### 1. RNA molecule

According to the first embodiment, the present invention relates to an RNA molecule. The RNA molecule includes:
(a) a recognition region that specifically recognizes a target substance;
(b) a cap-independent translational activation region positioned on the 3' terminal side of the recognition region; and
(c) a coding region positioned on the 3' terminal side of the translational activation region, the coding region being translated by the translational activation region, in which
   the RNA molecule does not contain a 5' cap structure.

The RNA molecule may optionally include the following:
(d) a spacer region positioned on the 5' side of the recognition region.

The RNA molecule according to the present embodiment is typically mRNA that is translated into a protein encoded by the coding region in the presence of a target substance and is translationally repressed in the absence of a target substance. The RNA molecule may include both of *in vitro* synthesized mRNA and mRNA prepared as an artificial plasmid and produced by transcription in cells. In the present specification, the RNA molecule according to the present embodiment is referred to as ON switch mRNA. The ON switch mRNA may also be abbreviated as "ON switch" or "switch mRNA". The state in which the RNA molecule according to the present embodiment can be translated into a protein encoded by the coding region in the absence of a target substance is referred to as an ON state. Typically, the case in which the target substance is present in a form capable of binding to the RNA molecule is referred to as an ON state. On the other hand, the state in which the RNA molecule cannot be translated into a protein is referred to as an OFF state. Typically, the case in which the target substance is absent or the target substance is not present in a form capable of binding to the RNA molecule is referred to as an OFF state.

The target substance of the RNA molecule according to the present embodiment refers to a substance that is present in a target cell when the RNA molecule according to the present embodiment undergoes ON-OFF regulation. In a certain embodiment, the target substance can refer to a substance that is present in the cytoplasm or the nucleus of a cell. The target substance may be an endogenous cellular substance and may be a metabolite resulting from a natural or artificial intracellular reaction. The target substance may be a substance artificially introduced to the inside of a cell from the outside of the cell, or a substance taken into the inside of a cell from the outside of the cell through a biological function. Examples of the target substance include, but are not limited to, proteins, peptides, nucleic acids, synthetic or natural low-molecular compounds, complexes containing metal ions, and complexes, fragments, and metabolites of these substances. Examples of the complex include complexes of miRNA and a protein, protein groups such as p53 related to a disease, transcriptional factor complexes that regulate cell fate, noncoding RNA-protein complexes, and viral particles. In the present specification, the target substance is also referred to as a ligand.

The structure and design of the RNA molecule according to the present embodiment will be described. The RNA molecule is a nucleic acid molecule that includes (a) a recognition region, (b) a translational activation region, and (c) a coding region, and does not contain a 5' cap structure.

### (a) Recognition region

The recognition region contained in the RNA molecule is a region comprising a nucleic acid sequence that specifically recognizes a target substance. The recognition region can form specific binding with a target substance in the presence of the target substance and protect the 5' end of the RNA molecule from degradation by exoribonuclease. Examples of the nucleic acid sequence that specifically recognizes a target substance include, but are not limited to, protein-binding RNA sequences such as Cas family protein-binding RNA, aptamers, aptazyme, and nucleic acid sequences that specifically recognize RNA including small RNA.

An aptamer, which is one example of a recognition region, refers to a nucleic acid that is selected so as to specifically recognize a target substance. Examples of the target substance of the aptamer include, but are not limited to, proteins, peptides, organic small molecules, cells, tissues, nucleic acids, viruses, and metal ions. The aptamer can be selected and designed by, for example, SELEX (systematic evolution of ligands by exponential enrichment), provided that the target substance is determined. Alternatively, the aptamer may be an aptamer that recognizes a particular target substance or an aptamer having a modified sequence capable of specifically recognizing a target substance, known from the literature or a database (e.g., https://www.aptagen.com/apta-index/). Alternatively, a database on naturally occurring RNA-binding proteins and their binding sequences (e.g., http://cisbp-rna.ccbr.utoronto.ca/) may be used.

The aptazyme, another example of the recognition region, refers to a ribozyme or a nucleic acid enzyme having a property of altering its own conformation or ribozyme activity by specifically recognizing a target substance. The aptazyme typically includes an aptamer domain and a ribozyme domain that exhibits catalytic action. The aptazyme is preferably an aptazyme that is conformationally altered by the binding of a target substance so that the 5' end of the RNA molecule according to the present embodiment can be blocked. If necessary, the aptazyme may be modified for use so as not to cause undesired catalytic action, by introducing a mutation to the domain that exhibits catalytic action. To obtain and design the aptazyme, the aptazyme can be selected and designed by SELEX (systematic evolution of ligands by exponential enrichment), as in the aptamer. Alternatively, the aptazyme can also be obtained by fusing the aptamer with a ribozyme in consideration of a relationship to an active site of the ribozyme.

Examples of a nucleic acid sequence that specifically recognizes RNA, a further alternative example of the recognition region, include a nucleic acid sequence targeted by miRNA constituting an RNA induced silencing complex (RISC). The nucleic acid sequence targeted by miRNA constituting RISC is also referred to as a miRNA target sequence or a target sequence of miRNA. The target sequence of miRNA is a nucleic acid sequence capable of specifically recognizing the miRNA. The target sequence preferably has a mismatch from a sequence completely complementary to the miRNA. A complex of RISC and the RNA molecule is thereby formed without the cleavage of the target sequence by the RISC so that the 5' end of the RNA molecule can be protected. The mismatch from a sequence completely complementary to the miRNA can be a mismatch to the extent that the cleaving activity of an RISC does not occur. Examples thereof include mismatches of 30%, 40%, 50%, 60%, or 70% of the whole target sequence. Examples of such a mismatch include, but are not particularly limited to, mismatches of 16 bases, 15 bases, 14 bases, 13 bases, 12 bases, 11 bases, 10 bases, 9 bases, 8 bases, and 7 bases. The combination of the target sequence with the RISC that forms a complex, without cleaving the RNA molecule, by binding to the RNA molecule so that the 5' end of the RNA molecule is blocked can also be determined by preliminary experimentation.

The nucleic acid sequence constituting the recognition region can be determined, particularly, in relation to a target substance. For example, a nucleic acid sequence is preferred which forms an exoribonuclease-resistant structure such as an xrRNA structure by binding to a target substance. For example, a structure is preferred which attains dissociation constant Kd of less than 1000 µM, preferably 100 µM or less, more preferably 100 nM or less, for a complex of the nucleic acid sequence contained in the recognition region and a target substance, and allows the complex of the nucleic acid sequence contained in the recognition region and the target substance to be maintained on the RNA molecule so as not to structurally expose the 5'-terminal moiety of the RNA molecule. This is because such a complex has highly protective action against degradation by an exoribonuclease. The recognition region recognizes a target substance and forms a complex therewith so that the 5' end of the mRNA molecule according to the present embodiment can be protected.

The recognition region may have one repeat of a nucleic acid sequence that is recognized by one type of a target substance, or it may have two or more repeats thereof. Alternatively, the recognition region may have two or more different nucleic acid sequences that are recognized by different target substances, respectively.

The recognition region is preferably positioned on the 5' side of the translational activation region given below. A nucleic acid sequence of one or more bases may be optionally contained between the recognition region and the translational activation region. The optionally contained nucleic acid sequence is not limited by its strand length as long as the sequence is not cleaved by an endogenous cellular substance or the like. The optionally contained nucleic acid sequence may be a sequence that functions merely as a spacer, or it may be a sequence having a function of promoting the ON-OFF shift of the switch constituted by the RNA molecule according to the present embodiment.

### (b) Translational activation region

The translational activation region is a region comprising a nucleic acid sequence capable of activating the coding region in a 5' cap-independent manner. The translational activation region can be, but is not limited to, a nucleic acid sequence comprising an internal ribosome entry site (IRES), a ribosome binding site (RBS), or a translational activator recognition site such as an aptamer sequence of a translational initiation factor. The IRES is desirably an IRES having activity in a cell of interest, or an IRES available for *in vitro* transcribed mRNA. The IRES may be a virus-derived IRES, or it may be a cellular mRNA-derived IRES. The nucleic acid sequence of the IRES can be obtained from a database (e.g., http://www.iresite.org/). Preferred examples of the virus-derived IRES include, but are not limited to, those from encephalomyocarditis virus (EMCV) or coxsackievirus B group type 3 (CVB3). Examples of the cellular mRNA-derived IRES include, but are not limited to, IRES residing on RPL41, GATA1, or HMGA1 mRNA.

The translational activation region is positioned on the 3' side of the recognition region (a) and is positioned on the 5' side of the coding region (c). In the case of, for example, using an IRES as the translational activation region, the IRES may contain a start codon in the IRES sequence, depending on the type of IRES. Thus, the translational activation region positioned on the 5' side of the coding region refers to the case in which at least the 5' end of the translational activation region is positioned on the 5' side of a start codon of the coding region (c). A nucleic acid sequence of one or more bases may be optionally contained between the translational activation region and the start codon. The optionally contained nucleic acid sequence may be a sequence that functions merely as a spacer, or it may be a sequence having a function of promoting the ON-OFF shift of the switch constituted by the RNA molecule according to the present embodiment.

### (c) Coding region

The coding region is a region comprising a nucleic acid sequence that is translated by the translational activation region, and it is typically a region comprising a nucleic acid sequence encoding a protein. More specifically, the coding region includes a start codon, a nucleic acid sequence encoding a protein and a stop codon, in this order, from the 5' side. In this context, the "protein" includes a fragment of the protein, a fusion protein, and a peptide.

The protein can be a freely selected protein and is not limited by its type or number. A desired protein can be included therein according to the use and purpose of the RNA molecule. The protein may include, for example, a detectable protein, a therapeutic protein, and an RNA-binding protein capable of binding to another ON switch or OFF switch mRNA. The detectable protein, the therapeutic protein, or the RNA-binding protein is not limited to a protein that exerts a detection, therapeutic, or binding function in itself, and may be a substance that exerts the detection, therapeutic, or binding function together with a substance other than the protein encoded by the RNA molecule.

The detectable protein refers to a freely selected protein capable of displaying detectable information after being translated. The detectable protein can be a protein that can be visualized and quantified by developing fluorescence, luminescence, or color, or by assisting in developing fluorescence, luminescence, or color, for example.

Examples of a fluorescent protein include, but are not limited to: blue fluorescent proteins such as Sirius and EBFP; cyan fluorescent proteins such as mTurquoise, TagCFP, AmCyan, mTFP1, MidoriishiCyan, and CFP; green fluorescent proteins such as TurboGFP, AcGFP, TagGFP, Azami-Green (e.g., hmAG1), ZsGreen, EmGFP, EGFP, GFP2, and HyPer; yellow fluorescent proteins such as TagYFP, EYFP, Venus, YFP, PhiYFP, PhiYFP-m, TurboYFP, ZsYellow, and mBanana; orange fluorescent proteins such as KusabiraOrange (e.g., hmKO2) and mOrange; red fluorescent proteins such as TurboRFP, DsRed-Express, DsRed2, TagRFP, DsRed-Monomer, AsRed2, and mStrawberry; and near-infrared fluorescent proteins such as TurboFP602, mRFP1, JRed, KillerRed, mCherry, HcRed, KeimaRed (e.g., hdKeimaRed), mRasberry, and mPlum. Examples of the luminescent protein can include, but are not limited to, aequorin.

Examples of a protein that assists in developing fluorescence, luminescence, or color can include, but are not limited to, enzymes, such as luciferase, phosphatase, peroxidase, and β lactamase, which degrade a fluorescent, luminescent, or color precursor. For use of an RNA molecule comprising, in the coding region, a nucleic acid sequence encoding the protein that assists in developing fluorescence, luminescence, or color, it is necessary to use the RNA molecule in a form that allows the corresponding precursor to make contact with the protein produced by translation from the RNA molecule. For example, the precursor can be brought into contact with a cell harboring the RNA molecule, or the corresponding precursor can be introduced to a cell harboring the RNA molecule.

The therapeutic protein refers to a protein that can be used in the treatment, prevention, or diagnosis of a disease or a condition by influencing the function of a cell. The influence on the function of a cell includes elevation or reduction in the predetermined function of the cell or maintenance of the predetermined function in a constant range. Examples of the therapeutic protein can include, but are not limited to, cell proliferative proteins, cell-killing proteins, cell signaling factors, drug resistance genes, transcriptional regulation factors, translational regulation factors, differentiation regulation factors, reprogramming inducers, RNA-binding protein factors, chromatin regulation factors, membrane proteins, and fragments and complexes of these proteins. These proteins can be interpreted as being capable of displaying detectable information by influencing the function of a cell, and can thus be regarded as both therapeutic proteins and detectable proteins. Other examples of the therapeutic protein can include, but are not limited to, enzymes, growth factors, antibodies, antigens, proteins constituting viruses or their moieties, proteins that inhibit viral production, genome editing proteins, and fragments and complexes of these proteins.

For example, the cell proliferative protein allows only a cell expressing this protein to proliferate, and functions as a marker by identifying the proliferated cell. The cell-killing protein kills a cell itself that contains or does not contain a particular molecule (target substance) by causing the cell death of the cell expressing this protein, and functions as a marker that indicates the life or death of the cell. The cell signaling factor allows a cell expressing this protein to emit a particular biological signal, and functions as a marker by identifying this signal. Examples of the cell-killing protein include, but are not limited to, RNases such as *Bacillus amyloliquefaciens-*derived barnase, toxins such as HokB, Fst, GhoT (membrane disruption), HipA (inhibition of nucleic acid elongation by phosphorylation), RelE, YafO, VapC, MazF, MqsR, PemKHicA (endonuclease), FicT (adenylation), oc (phosphorylation), CcdB, ParE (gyrase inhibitor), Tact (inhibitor of translation), and cbtA (inhibitor of cytoskeletal protein), and apoptosis-inducing proteins such as Bax and Bim. The translational regulation factor, as one example, functions as a marker by recognizing and binding to the tertiary structure of particular RNA and thereby regulating the translation of another mRNA into a protein. Examples of the translational regulation factor include 5R1, 5R2 (Nat Struct Biol. 1998 Jul; 5 (7): 543-6), B2 (Nat Struct Mol Biol. 2005 Nov; 12 (11): 952-7), Fox-1 (EMBO J. 2006 Jan 11; 25 (1): 163-73.), GLD-1 (J Mol Biol. 2005 Feb 11; 346 (1): 91-104.), Hfq (EMBO J. 2004 Jan 28; 23 (2): 396-405), HuD (Nat Struct Biol. 2001 Feb; 8 (2): 141-5.), SRP19 (RNA. 2005 Jul; 11 (7): 1043-50), L1 (Nat Struct Biol. 2003 Feb; 10 (2): 104-8.), L11 (Nat Struct Biol. 2000 Oct; 7 (10): 834-7.), L18 (Biochem J. 2002 Mar 15; 362 (Pt 3): 553-60), L20 (J Biol Chem. 2003 Sep 19; 278 (38): 36522-30.), L23 (J Biomol NMR. 2003 Jun; 26 (2): 131-7), L25 (EMBO J. 1999 Nov 15; 18 (22): 6508-21.), L30 (Nat Struct Biol. 1999 Dec; 6 (12): 1081-3.), LicT (EMBO J. 2002 Apr 15; 21 (8): 1987-97.), MS2 coat (FEBS J. 2006 Apr; 273 (7): 1463-75.), Nova-2 (Cell. 2000 Feb 4; 100 (3): 323-32), Nucleocapsid (J Mol Biol. 2000 Aug 11; 301 (2): 491-511.), Nucleolin (EMBO J. 2000 Dec 15; 19 (24): 6870-81.), p19 (Cell. 2003 Dec 26; 115 (7): 799-811), L7Ae (RNA. 2005 Aug; 11 (8): 1192-200.), PAZ (PiWi Argonaut and Zwille) (Nat Struct Biol. 2003 Dec; 10 (12): 1026-32.), RNaseIII (Cell. 2006 Jan 27; 124 (2): 355-66), RR1-38 (Nat Struct Biol. 1998 Jul; 5 (7): 543-6.), S15 (EMBO J. 2003 Apr 15; 22 (8): 1898-908.), S4 (J Biol Chem. 1979 Mar 25; 254 (6): 1775-7.), S8 (J Mol Biol. 2001 Aug 10; 311 (2): 311-24.), SacY (EMBO J. 1997 Aug 15; 16 (16): 5019-29.), SmpB (J Biochem (Tokyo). 2005 Dec; 138 (6): 729-39), snRNP U1A (Nat Struct Biol. 2000 Oct; 7 (10): 834-7.), SRP54 (RNA. 2005 Jul; 11 (7): 1043-50), Tat (Nucleic Acids Res. 1996 Oct 15; 24 (20): 3974-81.), ThrRS (Nat Struct Biol. 2002 May; 9 (5): 343-7.), TISlld (Nat Struct Mol Biol. 2004 Mar; 11 (3): 257-64.), Virp1 (Nucleic Acids Res. 2003 Oct 1; 31 (19): 5534-43.), VtslP (Nat Struct Mol Biol. 2006 Feb; 13 (2): 177-8), and λN (Cell. 1998 Apr 17; 93 (2): 289-99).

The coding region may include a nucleic acid sequence encoding a fusion protein consisting of a combination of two or more of the proteins listed above. The coding region may include a self-cleavage sequence between the two or more proteins and may thereby be configured such that the translated proteins function as separate protein molecules.

### (d) Spacer region

The RNA molecule may include a spacer region as an optional region on the 5' side of the recognition region (a). The spacer region can be constituted by a freely selected nucleic acid sequence on the order of 1 to 100 bases. The nucleic acid sequence constituting the spacer region preferably has substantially no specificity for a target substance. The spacer region thus provided can improve the translation efficiency of the switch by the RNA molecule.

According to one embodiment of the present invention, in the RNA molecule, the recognition region (a) may be linked to the 5' side of the translational activation region (b). In this context, the term "linked" means that respective regions are bonded directly to each other without containing an extra nucleic acid between the respective regions. According to one embodiment of the present invention, in the RNA molecule, the translational activation region (b) may be linked to the 5' side of the coding region (c), and the spacer region (d) may be linked to the 5' side of the recognition region (a).

The RNA molecule according to the present embodiment typically includes 5'-UTR, the coding region, and 3'-UTR in the 5'-to-3' direction. The 5'-UTR includes the recognition region and the translational activation region in the 5'-to-3' direction. Preferably, the 5'-UTR includes the spacer region, the recognition region, and the translational activation region in the 5'-to-3' direction. The 3'-UTR resides on the 3' side of the coding region and includes a poly(A) tail. The poly(A) tail can have a total A length of 50-mer or more and may contain a nucleobase other than A in the poly(A) sequence.

The RNA molecule according to the present embodiment does not contain a 5' cap structure. The 5' cap structure refers to a modified structure that resides at the 5' end of an RNA molecule and is recognized by a translational initiation factor eIF4E. Known examples of the 5' cap structure include, but are not limited to, 7-methylguanosine 5'-phosphate (cap structure), Anti-Reverse Cap Analog (ARCA) from Ambion, Inc., m7G(5')ppp(5')G RNA Cap Structure Analog from New England Biolabs Inc., and CleanCap from TriLink BioTechnologies. By not containing the 5' cap structure, the 5'-terminal structure of the RNA molecule according to the present embodiment can be a nucleobase constituting the spacer region or a nucleobase constituting the recognition region, and can be a nucleic acid having usual adenine, guanine, cytosine, uracil, or a modified base mentioned later without impairing an effect of RNA degradation and translation and/or detection related to the drive of the RNA molecule according to the present embodiment as a switch.

The RNA molecule according to the present embodiment may be modified at a sugar residue (ribose) of each nucleotide for the purpose of, for example, reducing cytotoxicity, unless such a modification impairs an effect of RNA degradation and translation and/or detection related to the drive of the RNA molecule according to the present embodiment as a switch. Examples of the site modified in the sugar residue include a hydroxy group or a hydrogen atom at position 2', 3', and/or 4' of the sugar residue replaced with another atom. Examples of the type of the modification include fluorination, alkoxylation (e.g., methoxylation and ethoxylation), O-allylation, S-alkylation (e.g., S-methylation and S-ethylation), S-allylation, and amination (e.g., -NH₂). Such a modification of the sugar residue can be performed by a method known per se in the art (see, for example, Sproat et al., (1991) Nucl. Acid. Res. 19, 733-738; Cotton, et al., (1991) Nucl. Acid. Res. 19, 2629-2635; and Hobbs, et al., (1973) Biochemistry 12, 5138-5145).

The sugar residue of the RNA molecule may take the form of BNA (bridged nucleic acid (LNA: linked nucleic acid)) in which a bridged structure is formed at positions 2' and 4', unless such bridge formation impairs an effect of RNA degradation and translation and/or detection related to the drive of the RNA molecule according to the present embodiment as a switch. Such a modification of the sugar residue can also be performed by a method known per se in the art (see, for example, Tetrahedron Lett., 38, 8735-8738 (1997); Tetrahedron, 59, 5123-5128 (2003); and Rahman S.M.A., Seki S., Obika S., Yoshikawa H., Miyashita K., Imanishi T., J. Am. Chem. Soc., 130, 4886-4896 (2008)). The mRNA molecule may be modified (e.g., chemically substituted) at a nucleobase (e.g., purine or pyrimidine). Examples of such a modification include modification of pyrimidine at position 5, modification of purine at position 6 and/or 8, modification at exocyclic amine, substitution at 4-thiouridine, and substitution at 5-bromo- or 5-iodo-uracil. A modified base such as pseudouridine (Ψ), N1-methylpseudouridine (N1mΨ), or 5-methylcytidine (5mC) may be contained instead of usual uridine or cytidine for the purpose of, for example, reducing cytotoxicity. The position of the modified base in the case of either uridine or cytidine may be independently at the position of any one of the bases or some of the bases. In the case of some of the bases, the modified base can be randomly positioned and be at a freely selected ratio.

A phosphate group (e.g., a terminal phosphate residue) contained in the RNA molecule may be further modified in order to enhance, for example, resistance to nuclease and hydrolysis, unless the further modification impairs an effect of RNA degradation and translation and/or detection related to the drive of the RNA molecule according to the present embodiment as a switch. For example, a P(O)O group which is a phosphate group may be substituted by P(O)S (thioate), P(S)S (dithioate), P(O)NR₂ (amidate), P(O)R, R(O)OR', CO or CH₂ (formacetal), or 3'-amine (-NH-CH₂-CH₂-) [wherein each R or R' is independently H or substituted or unsubstituted alkyl (e.g., methyl or ethyl)]. Examples of the linking group include -O-, -N-, and S-. Through such a linking group, the group is capable of binding to an adjacent nucleotide.

One skilled in the art can synthesize the RNA molecule according to the present embodiment by a freely selected known genetic engineering method, provided that its molecular structure and nucleic acid sequence are determined in accordance with the description above. According to a certain embodiment, the RNA molecule according to the present embodiment can be obtained as a synthetic RNA molecule by an *in vitro* synthesis method using template DNA containing a promoter sequence as a template. The RNA molecule that does not contain a 5' cap structure can be produced without carrying out the operation of capping in a usual method for synthetic mRNA molecules. One of the advantages of the present invention is that the synthetic RNA molecule can be obtained as designed by a convenient method. In the case of preparing the RNA molecule as a synthetic RNA molecule, the 5' end of the RNA molecule preferably assumes a monophosphorylated structure. This is because the synthetic RNA molecule, when introduced to a cell, prevents leaky expression in the absence of a target substance. Examples of the approach of allowing the 5' end of the RNA molecule to assume a monophosphorylated structure include an approach of subjecting an RNA molecule after transcription in an *in vitro* synthesis method to dephosphorylation treatment with bacterial RNA 5' pyrophosphohydrolase (RppH), an approach of using GMP at the time of transcription, and a combination thereof. In addition, the 5' end of the RNA molecule can be monophosphorylated by a usual method known to one skilled in the art.

According to another embodiment, the RNA molecule according to the present embodiment may be intracellularly biosynthesized by introducing an expression vector serving as a DNA construct encoding the nucleic acid sequence of the RNA molecule to a cell. A common vector well known in the art can be used as the expression vector encoding the sequence of the RNA molecule. Examples thereof include virus vectors, artificial chromosome vectors, plasmid vectors, and expression systems using a transposon (also called transposon vectors). Examples of the virus vector include retrovirus vectors, lentivirus vectors, adenovirus vectors, adeno-associated virus vectors, and Sendai virus vectors. Examples of the artificial chromosome vector include one of a human artificial chromosome (HAC), a yeast artificial chromosome (YAC), and a bacterial artificial chromosome (BAC and PAC). A general plasmid for mammals can be used as the plasmid vector. The plasmid vector may be, for example, an episomal vector. Examples of the transposon vector include expression vectors using a piggyBac transposon.

The vector may be a vector encoding only the nucleic acid sequence of the RNA molecule according to the present embodiment. In this case, the RNA molecule according to the present embodiment can be obtained by removing a 5' cap structure from an intracellularly biosynthesized RNA molecule having the 5' cap structure. Examples of a substance that can remove the 5' cap structure from the intracellularly biosynthesized RNA molecule include, but are not limited to, ribozymes, RNA-degrading sequences, RISC complexes, decapping enzymes, and RNA-targeting Cas proteins. Examples of the RNA-degrading sequence include target sequences of endoribonuclease, such as Csy4 target sequences, although this is not limited to particular sequences. In the present specification, the substance that can remove the 5' cap structure is referred to as a 5' cap structure-removing substance, and a nucleic acid sequence encoding the 5' cap structure-removing substance is referred to as a 5' cap structure-removing sequence. Specific examples of the 5' cap structure-removing sequence include, but are not limited to, self-cleaving ribozymes, miRNA target sequences, target sequences of RNase P or Z, and RNA-degrading sequences. The 5' cap structure-removing sequence can be a sequence encoding a protein that can remove the 5' cap structure. The 5' cap structure can be removed from the intracellularly biosynthesized RNA molecule by introducing the 5' cap structure-removing substance to the cell. Examples of the introduction method include vectors encoding the nucleic acid sequence encoding the 5' cap structure-removing substance. Examples of the self-cleaving ribozyme include, but are not limited to, N79 which is a modified form of *Schistosoma mansoni-*derived ribozyme Sm1, hammerhead ribozyme (HHR) which is a modified form of tobacco ringspot virus-derived ribozyme, and their derivatives and mutants that retain self-cleaving activity. The derivatives and the mutants that retain the self-cleaving activity of the self-cleaving ribozyme can maintain, for example, the structure and the sequence of a catalytic core of freely selected self-cleaving ribozyme and assume a consensus structure as the whole structure of the ribozyme.

For example, a derivative of HHR in FIG. 11 may be, for example, a derivative having stem-loop structures at two sites, which is obtained by introducing a loop (e.g., a loop that connects base 1 and base 49 and is constituted by four or more bases (preferably four to eight bases)) to the end of a stem structure formed from bases 1 to 6 and bases 44 to 49 to form a new stem-loop, and cleaving the loop site of a stem loop positioned at bases 29 to 42. The cleavage position is not particularly limited as long as base pairs between 29 to 33 (ACGAG) and 38 to 42 (CUCGU) are retained. In particular, as for HHR, the sequence of the stem moiety can be constituted, as shown in FIG. 11, by freely selected base pairs as long as the stem is formed. The HHR may be a molecule having the RNA sequence represented by SEQ ID NO: 49, or a molecule modified such that the number of base pairs in the stem moiety is 5 base pairs, 7 base pairs, 8 base pairs, or 9 or more base pairs. The derivative may also be modified such that the number of base pairs in the stem moiety formed from bases 1 to 6 and bases 44 to 49 is, for example, 4 base pairs, 5 base pairs, 7 base pairs, 8 base pairs, or 9 or more base pairs.

Preferably, the RNA molecule according to the present embodiment can be efficiently intracellularly produced by introducing a vector comprising the 5' cap structure-removing sequence and a sequence encoding the nucleic acid sequence of the RNA molecule to a cell. The vector preferably includes a promoter sequence, the 5' cap structure-removing sequence, and the sequence encoding the nucleic acid sequence of the RNA molecule in the 5'-to-3' direction. Use of such a vector enables the RNA molecule according to the present embodiment to be intracellularly produced by cleaving off the 5' cap site by the 5' cap structure-removing substance such as a ribozyme after transcription of RNA. One vector having both the 5' cap structure-removing sequence and the sequence encoding the nucleic acid sequence of the RNA molecule has advantages that the switch can be introduced into cells without depending on *in vitro* transcribed RNA; and complicated conditioning can be performed by the drive of the switch.

### 2. RNA molecule

According to the second embodiment, the present invention relates to an RNA molecule. The RNA molecule includes:
(A) a recognition region that specifically recognizes a target substance; and
(B) a reporter region positioned on the 3' terminal side of the recognition region, in which
   the RNA molecule does not contain a 5' cap structure.

Unlike the RNA molecule according to the first embodiment, the RNA molecule according to the present embodiment is an RNA capable of being switched ON-OFF without involving a translational function. The RNA molecule may include both *in vitro* synthesized RNA and RNA that has been artificially prepared as a plasmid and produced by transcription in cells. The RNA molecule according to the present embodiment is also referred to as ON switch RNA and is also referred to as a complex ON switch to distinguish it from the RNA molecule of the first embodiment. The state in which the RNA molecule according to the present embodiment is placed under conditions involving a target substance and exoribonuclease so that the target substance binds to the RNA molecule to inhibit degradation by exoribonuclease, whereas the reporter region can form a complex with a substrate mentioned later to generate an optical signal is referred to as an ON state. On the other hand, the state in which the RNA molecule is degraded by exoribonuclease is referred to as an OFF state. Typically, the case in which the target substance is absent or the target substance is not in a form capable of inhibiting the degradation of the RNA molecule by exoribonuclease is referred to as an OFF state.

The target substance of the RNA molecule according to the present embodiment can be designed from the same options as those of the target substance of the RNA molecule according to the first embodiment. However, the RNA molecule according to the present embodiment is used as a complex ON switch without limitations to intracellular usage. Hence, a substance not normally present in a cell, for example, a heavy metal such as Co²⁺ or Cd²⁺, or an environmental pollutant such as 2,4-dinitrotoluene, can be used as a target substance.

Next, the structure and design of the RNA molecule according to the present embodiment will be described. The RNA molecule is a nucleic acid molecule that includes (A) a recognition region and (B) a reporter region, and does not contain a 5' cap structure. Due to a lack of a translational function, it is preferred that the RNA molecule not include a translational activation region, a coding region encoding a protein, a poly(A) structure, or the like.

The recognition region (A) according to the present embodiment can be configured in the same manner as in the recognition region (a) in the RNA molecule according to the first embodiment, and can be selected and designed so as to adapt to a target substance.

The reporter region (B) is typically constituted by a nucleic acid sequence comprising a particular light-up aptamer that generates an optical signal by binding to an RNA probe. Typically, a light-up aptamer can be used, which emits fluorescence by binding to a particular RNA probe (substrate) and is a nucleic acid sequence also called fluorescent RNA aptamer. Examples of the light-up aptamer include, but are not limited to, Spinach aptamer which emits green fluorescence (Nat. Chem. Biol. 10686-691), and its modified forms (Nat. Struct. Mol. Biol. 21658-663), Broccoli aptamer which emits green fluorescence (J. Am. Chem. Soc. 2014, 136, 46, 16299-16308), Corn aptamer which emits yellow fluorescence (Nat. Chem. Biol. 131195-1201), Mango-II aptamer which emits orange fluorescence (Biochemistry 573544-3548), and DIR2s aptamer which emits red fluorescence (Nat. Commun. 94542-4542).

The RNA molecule according to the present embodiment is a nucleic acid molecule that does not contain a 5' cap structure, as in the RNA molecule according to the first embodiment. The definition of not containing a 5' cap structure is as described in the first embodiment. Thus, the 5'-terminal structure of the RNA molecule according to the present embodiment can be the same as that of the RNA molecule according to the first embodiment.

The recognition region (A) and the reporter region (B) may be linked to each other, or a nucleic acid sequence of one more bases may be optionally contained between these regions. The optionally contained nucleic acid sequence may be a sequence that functions merely as a spacer or may be a sequence having other functions, and it is preferably a sequence that is difficult to cleave. A spacer region may or may not be contained on the 5' side of the recognition region (A), as in the RNA molecule according to the first embodiment. A nucleic acid sequence of one or more bases may or may not be optionally contained on the 3' side of the reporter region (B).

The RNA molecule according to the present embodiment can be designed and produced in the same manner as in the RNA molecule according to the first embodiment. A sugar residue may be modified, and a phosphate group may be modified, unless such modification impairs an effect of RNA degradation and optical signal generation related to the drive of the RNA molecule of the present embodiment. The RNA molecule according to the present embodiment can be obtained as a synthetic RNA molecule by an *in vitro* synthesis method. In this case, the RNA molecule is preferably produced such that the 5' end of the RNA molecule assumes a monophosphorylated structure. The RNA molecule according to the present embodiment may be intracellularly biosynthesized by introducing an expression vector serving as a DNA construct to a cell. In this case as well, the RNA molecule can be biosynthesized in a form in which a 5' cap structure is removed from an RNA molecule having the 5' cap structure by the method described in the first embodiment. In another embodiment, a DNA vector for non-mRNA expression containing U6 promoter or the like may be used.

The RNA molecule according to the present embodiment is capable of being switched ON-OFF without using a translational mechanism. Hence, the RNA molecule can function as a complex ON switch that generates an optical signal by responding to a target substance in a wide range of sample solutions without being limited to being intracellular.

### 3. Translational regulation method and system

According to the third embodiment, the present invention relates to a translational regulation method and system. In particular, the present invention relates to a translational regulation method using the RNA molecule or the DNA construct according to the first embodiment. The translational regulation method according to the third embodiment includes the step of bringing the RNA molecule according to the first embodiment into contact with a cell or the step of introducing the DNA construct according to the first embodiment to a cell.

In the present specification, the cell is not particularly limited and can be a freely selected cell. The cell may be a single cell or may be a cell population which is a grouping of two or more cells. The cell population theoretically has no upper limit on the number of cells and refers to a population consisting of a freely selected number of cells. The cell population can be, for example, a cell population that can include multiple types of different cells. The cell population can be, in particular a cell population that can include cells that may contain a target substance that specifically recognizes the recognition region of the RNA molecule.

The cell may be a cell collected from a species of unicellular organism, or a species of multicellular organism, or it may be a further artificially manipulated cell (including a cell line). For example, yeast, insect cells, or animal cells may be used. Among these, animal cells are preferred. Examples of the animal cells include cells derived from mammals (e.g., mice, rats, hamsters, guinea pigs, dogs, monkeys, orangutans, chimpanzees, and humans). The cells derived from a mammal may be, for example, a cell line such as monkey COS-7 cells, monkey Vero cells, Chinese hamster ovary (CHO) cells, dhfr gene-deficient CHO cells, mouse L cells, mouse AtT-20 cells, mouse myeloma cells, rat GH3 cells, human embryonic kidney-derived cells (e.g., HEK293 cells), human liver cancer-derived cells (e.g., HepG2), or human FL cells. Primary cultured cells prepared from the tissues of a human and other mammals can be used. Alternatively, zebrafish embryos, Xenopus oocytes, and the like may be used.

The degree of differentiation of the cell, the age of the animal from which the cell is collected, and the like is not particularly limited. The cell may be any of (A) stem cells, (B) progenitor cells, (C) somatic cells in final differentiated form, and (D) other cells. Examples of the stem cells (A) include, but are not limited to, embryonic stem (ES) cells, clone embryo-derived embryonic stem (ntES) cells obtained by nuclear transplantation, sperm stem cells ("GS cells"), embryonic germ cells ("EG cells"), and induced pluripotent stem (iPS) cells. Examples of the progenitor cells (B) include tissue stem cells (somatic stem cells) such as neural stem cells, hematopoietic stem cells, mesenchymal stem cells, and dental pulp stem cells. Examples of the somatic cells (C) include keratinized epithelial cells (e.g., keratinized epidermal cells), mucosal epithelial cells (e.g., epithelial cells on the tongue surface), exocrine epithelial cells (e.g., mammary gland cells), hormone-secreting cells (e.g., adrenomedullary cells), cells for metabolism and/or storage (e.g., hepatocytes), luminal epithelial cells constituting the interface (e.g., type I pneumocytes), luminal epithelial cells of internal tubules (e.g., vascular endothelial cells), ciliated cells having a carrying capacity (e.g., airway epithelial cells), cells for secretions of extracellular matrix (e.g., fibroblasts), contractile cells (e.g., smooth muscle cells), cells of the blood and the immune system (e.g., T lymphocytes), cells related to sensory reception (e.g., rod cells), neurons and glial cells of the central or peripheral nervous system (e.g., astrocytes), pigment cells (e.g., retinal pigment epithelial cells), and their progenitor cells (tissue progenitor cells). Examples of other cells (D) include cells that have undergone induction of differentiation. Progenitor cells and somatic cells obtained by the induction of differentiation from pluripotent stem cells are also included therein. Cells induced by so-called "direct conversion (also referred to as direct reprogramming or trans-differentiation)" in which somatic cells or progenitor cells differentiate directly into desired cells without undergoing an immature state may be used.

The step of bringing the RNA molecule into contact with a cell can be carried out by a typical method known to one skilled in the art. The RNA molecule may be brought into contact with a cell and thereby introduced into the cell. The method for introducing the RNA molecule directly to a cell can be a method of introducing the RNA molecule to a cell *in vitro* or *in vivo.* Examples of the *in vitro* RNA molecule introduction method include, but are not limited to, lipofection, a liposome method, electroporation, a calcium phosphate coprecipitation method, a DEAE dextran method, microinjection, and a gene gun method. Advantages brought about by the introduction of the synthetic RNA molecule include the absence of integration into the genome and easy availability of cells harboring the RNA molecule in medical applications. Examples of the *in vivo* RNA molecule introduction method include, but are not limited to, intramuscular injection, subcutaneous injection, intravenous injection, and intra-articular injection for mammals.

The step of introducing the DNA construct to a cell mainly includes the same method as that for the *in vitro* introduction of the RNA molecule. A DNA construct, such as an episomal vector, by which a transgene is not integrated into the genome of the recipient cell can be introduced to a cell by the same method as that for the *in vivo* introduction of the RNA molecule.

When the RNA molecule according to the first embodiment or the DNA construct encoding this RNA molecule is introduced into a cell, the translation of a protein encoded by the coding region is activated in response to a target substance. Specifically, a target substance binds to the RNA molecule, provided that the target substance in the cell is present in a form capable of binding to the recognition region of the RNA molecule. The RNA molecule is thereby protected from degradation by exoribonuclease and is translated into the protein encoded by the coding region. On the other hand, the RNA molecule undergoes degradation by exoribonuclease, provided that the target substance is absent in the cell or the target substance is not present in a form capable of binding to the recognition region of the RNA molecule. Hence, the translation of the protein encoded by the coding region is inhibited.

The translational regulation method according to the present embodiment may include the step of detecting the translation of a protein encoded by the coding region after the contact or introduction step. The detection step may differ depending on the type of protein encoded by the coding region. Examples of the detection apparatus include, but are not limited to, flow cytometers, imaging cytometers, fluorescence microscopes, luminescence microscopes, and CCD cameras.

One skilled in the art can use a suitable apparatus as such a detection apparatus, depending on the type or form of the protein encoded by the coding region. When the protein encoded by the coding region is, for example, a fluorescent protein or a luminescent protein, the protein can be quantified using a detection apparatus such as a flow cytometer, an imaging cytometer, a fluorescence microscope, or a CCD camera. When the protein encoded by the coding region is a protein that assists in developing fluorescence, luminescence, or color, a possible method for quantifying the protein employs a detection apparatus such as a luminescence microscope, a CCD camera, or a luminometer. When the protein encoded by the coding region is a membrane-localized protein, a possible method for quantifying the detectable protein employs a cell surface protein-specific detection reagent such as an antibody, and the detection apparatus described above. In addition, the expression of the protein can be detected by isolating cells, without a protein quantification process, using a magnetic cell separation apparatus (MACS), for example. When the protein encoded by the coding region is a drug resistance gene, the expression of the protein can be detected by isolating surviving cells by the administration of the drug. When the protein encoded by the coding region is a therapeutic protein, the expression of the protein can be detected by confirming a particular function of cells that can be regulated by the therapeutic protein. The particular function that can be confirmed may include the activity of cells and the life or death of cells.

The translational regulation method according to the present embodiment is capable of regulating the translation of a protein encoded by the RNA molecule in response to an intracellular target substance, by bringing the RNA molecule or the DNA construct according to the first embodiment into contact with a cell or a cell population and/or introducing the RNA molecule or the DNA construct according to the first embodiment into a cell or a cell population. The translational regulation method according to the present embodiment is capable of regulating the translation of the protein in a manner that is dependent, in particular, on the amount of the target substance, and is capable of detecting not only the presence or absence of the target substance but the amount of the target substance.

The translational regulation method according to the present embodiment may include the step of bringing at least two or more RNA molecules according to the first embodiment into contact with a cell. The translational regulation method can typically employ at least two or more different RNA molecule groups. In this context, one RNA molecule group refers to a gathering of a plurality of RNA molecules having the same structure. Each of the RNA molecules has the structural features described in the first embodiment. Specifically, each of the RNA molecule groups includes a plurality of RNA molecules each comprising: a recognition region that specifically recognizes a target substance; a cap-independent translational activation region positioned on the 3' terminal side of the recognition region; and a coding region positioned on the 3' terminal side of the translational activation region, the coding region being translated by the translational activation region, in which each of the RNA molecules does not contain a 5' cap structure. At least the target substance that is specifically recognized by the recognition region differs for each of the two or more different RNA molecule groups.

Thus, for example, each of first RNA molecules constituting a first RNA molecule group may have a first recognition region that specifically recognizes a first target substance, and each of second RNA molecules contained in a second RNA molecule group may have a second recognition region that specifically recognizes a second target substance. In such a case, the first target substance and the second target substance are different from each other. The translational activation region of the first RNA molecule and the translational activation region of the second RNA molecule may be the same or different and are preferably the same. The coding region of the first RNA molecule and the coding region of the second RNA molecule may be the same or different, depending on the usage thereof. The types of the different RNA molecule groups are theoretically not particularly limited and may be, for example, 3, 4, 5, 6, 7, or 8 or more types.

When these two or more RNA molecules or RNA molecule groups are brought into contact with a cell and introduced into the cell, the translation of a protein encoded by the coding region of each RNA molecule group can be activated or repressed in response to the presence of an intracellular target substance. Use of plural types of RNA molecule groups permits translational regulation that responds to a plurality of target substances. Although the RNA molecule is specifically described here, the DNA construct can also be translationally regulated in the same manner as above. In the case of using the DNA construct, two or more different RNA molecules may be produced as separate RNA molecules from a single vector.

The present embodiment provides a translational regulation system comprising one or two or more RNA molecules (groups) and/or DNA constructs (groups) for use in the translational regulation method. The translational regulation system may further include a nucleic acid or a drug for the detection of each RNA molecule (group) and/or DNA construct (group) protein and may include a reagent for use in introduction to cells or a medium for cell culture.

In a preferred aspect, the translational regulation system includes at least two RNA molecule groups, in which each of the RNA molecule groups includes a plurality of RNA molecules each comprising: a recognition region that specifically recognizes a target substance; a cap-independent translational activation region positioned on the 3' terminal side of the recognition region; and a coding region positioned on the 3' terminal side of the translational activation region, the coding region being translated by the translational activation region, in which each of the RNA molecules does not contain a 5' cap structure, and the target substance that is specifically recognized by the recognition region differs for each of the RNA molecule groups.

### 4. Method for detecting target substance

According to the fourth embodiment, the present invention relates to a method for detecting a target substance. The method for detecting a target substance according to the fourth embodiment can be a first aspect of detecting an intracellular target substance using the RNA molecule or the DNA construct according to the first embodiment, a second aspect of detecting a target substance in a cell-free gene expression system using the RNA molecule or the DNA construct according to the first embodiment, or a third aspect of detecting an intracellular or extracellular target substance using the RNA molecule or the DNA construct according to the second embodiment. Hereinafter, each form will be described.

### First aspect

The detection method according to the first aspect includes the step of bringing the RNA molecule according to the first embodiment into contact with a cell or a cell group or the step of introducing the DNA construct according to the first embodiment into a cell or a cell group. In the present aspect, the detection of a target substance refers to the confirmation of the presence of the target substance by using, as an index, the translation of a protein encoded by the coding region of the RNA molecule or the DNA construct according to the first embodiment. The confirmation of the presence of the target substance includes the confirmation of the presence or absence of the target substance and the quantification of the target substance. The confirmation of the presence or absence of the target substance and the quantification of the target substance include the display of visually recognizable information on the translated protein by the presence of the target substance. The visually recognizable information is not limited by direct generation of a visible signal by cells. The visually recognizable information is information obtained by converting a signal generated by cells to visually recognizable information through a numeric value, a chart, an image, or the like, and refers to information visually recognizable by one skilled in the art. The confirmation of the presence of the target substance may include selective survival, isolation, removal, or killing of cells containing the target substance or containing no target substance, and change in a particular activity.

In the method for detecting a target substance, the step of bringing the RNA molecule according to the first embodiment into contact with a cell or a cell group or the step of introducing the DNA construct according to the first embodiment into a cell or a cell group can be carried out by the same operation as that for the translational regulation method according to the third embodiment.

The method preferably further includes the step of detecting the translation of a protein encoded by the coding region of the RNA molecule after the contact or introduction step. In the detection step, the presence of the target substance can be confirmed in a desired form described in the translational regulation method according to the third embodiment. The present embodiment can also be interpreted as a composition for the detection of a target substance, comprising the RNA molecule or the DNA construct for use in the detection method.

The method for detecting a target substance is capable of detecting a freely selected target substance that can be present in a cell by the design of the recognition region of the RNA molecule. This permits, for example, detection of an intracellular disease-related protein and specific removal of diseased cells thereby, screening of cells based on intracellular protein expression, and diagnosis of an infectious disease by *in vitro* utilization.

### Second aspect

The method for detecting a target substance according to the second aspect includes the step of adding exoribonuclease and the RNA molecule according to the first embodiment or a DNA construct comprising a DNA sequence encoding the RNA molecule and a 5' cap structure-removing sequence to a cell-free gene expression system.

The present aspect differs from the first aspect in that the target substance is detected in a cell-free gene expression system without introducing the RNA molecule or the DNA construct into a cell; and the addition of exoribonuclease to the cell-free gene expression system is required.

The cell-free gene expression system refers to a composition that can cause extracellular expression of mRNA or DNA. For example, a mammalian, wheat, or *E. coli* cell lysate can be used in the cell-free gene expression system. The composition that may be used as the cell-free gene expression system is commercially available as an *in vitro* translation reagent, and such a commercially available reagent may be used. Alternatively, an artificial composition constituted by ribosomes, plural types of protein factors, amino acids, and buffer solution, for example, may be used. One example of the constituents of the artificial composition can include, but is not limited to, enzymes, *E. coli* ribosomes, aminoacyl tRNA synthetases (20 types), T7 RNA polymerase, and buffer solutions. Specific constituents contained in the cell-free gene expression system are described in detail in Shimizu et al., Methods 36 (2005) 299-304. One skilled in the art can construct the cell-free gene expression system on the basis of the literature. In the present aspect, the cell-free gene expression system can be mixed, for use, with a sample that may contain the target substance. The sample that may contain the target substance may be a liquid sample derived from a living body, such as urine or blood. The sample that may contain the target substance may be, for example, a cytotoxic substance or a water-source sample that may contain a harmful substance. The present aspect has the advantage that even a substance that is difficult to detect on the basis of intracellular translation because the substance may have an adverse effect on the function of cells can be used as a sample and can be detected.

The exoribonuclease can be XRN1, XRN2, archaebacterial RNase J, *E. coli* TrpH, or the like, although the exoribonuclease is not limited thereto.

The present aspect also preferably further includes the step of detecting the translation of a protein encoded by the coding region of the RNA molecule according to the first embodiment after the addition step. In the detection step, the presence of the target substance can be confirmed in a desired form described in the translational regulation method according to the third embodiment. The present aspect can also be interpreted as an invention that relates to a composition for the detection of a target substance, comprising the RNA molecule or the DNA construct for use in the detection method. The composition may include exoribonuclease.

The method for detecting a target substance according to the second aspect is capable of performing the same detection as that in the first aspect in a cell-free gene expression system without limitations to an intracellular manner. The present aspect also has the advantages that a cytotoxic substance can be detected; and a protein of interest can be produced *in vitro* in a manner dependent on conditions.

### Third aspect

The method for detecting a target substance according to the third aspect is a method for detecting a target substance, without a translational mechanism, using the RNA molecule according to the second embodiment. The method for detecting a target substance according to the third aspect includes the steps of:
(i) adding exoribonuclease, the RNA molecule according to the second embodiment or a DNA construct comprising a DNA sequence encoding the RNA molecule and a 5' cap structure-removing sequence, and an RNA probe that generates an optical signal by binding to the reporter region of the RNA molecule, to a sample; and
(ii) detecting the optical signal.

The method may optionally include the step of
(iii) adding bacterial RNA 5' pyrophosphohydrolase (RppH) to the sample.

The sample according to the present aspect can be a freely selected solution that may contain the target substance, and is not limited to cells or a cell-free gene expression system. However, a temperature condition, a pH condition, or the like is preferred which impairs neither the function of the exoribonuclease nor the signal-generating function of the RNA probe. In the case of adding RppH at the same time therewith, the solution is preferably placed under conditions that do not inhibit its enzymatic activity.

In the present aspect, the RNA molecule according to the second embodiment can be used as a switch for detection. When the sample is a cell or a cell-free gene expression system, a DNA construct comprising a DNA sequence encoding the RNA molecule and a 5' cap structure-removing sequence may be used.

In the step of adding, the components are mixed such that the RNA molecule according to the second embodiment is in a state that can be contacted with the target substance, the exoribonuclease, the RNA probe, and the optional RppH. The RppH can be used for monophosphorylating the 5' end of the RNA molecule, and may not have to be added if an RNA molecule having an already monophosphorylated 5' end is used. The exoribonuclease described in the second aspect can be used. The RNA probe is a substrate appropriate for the fluorescent RNA aptamer contained in the reporter region of the RNA molecule as described in the second embodiment, and a substrate that forms a complex with the fluorescent RNA aptamer to generate an optical signal can be used.

The order of addition of these substances is not particularly limited. These substances may be added at the same time or may be added separately. However, it is preferred that the target substance be present in the reaction system ahead of the enzymes mentioned below, and it is preferred that the RppH be added and brought into contact with the RNA molecule ahead of the addition of the exoribonuclease. This is because the RppH is added for the purpose of monophosphorylating the 5' end of the RNA molecule and preventing leaky expression of the complexed switch in an OFF state. This is also because in a cell-free system, 5'-terminal monophosphorylation is essential for degradation by the exoribonuclease XRN1 used. When the sample is a cell, the operation of adding the RNA molecule or the DNA construct to the sample can be performed in the same manner as in the first aspect of the present embodiment. The RNA probe may be added when the detection step is carried out.

In the step of detecting the optical signal, a detection apparatus or the like can be used to confirm the presence or absence of the optical signal or to quantify the optical signal. Examples of the detection apparatus include, but are not limited to, flow cytometers, imaging cytometers, fluorescence microscopes, luminescence microscopes, and CCD cameras, as in the third embodiment.

The mechanism of detection according to the present aspect will be described. In the presence of a target substance, the target substance binds to the recognition region of the RNA molecule according to the second embodiment so that the RNA molecule can be protected from degradation by the exoribonuclease. Hence, the fluorescent RNA aptamer residing on the 3' side of the recognition region of the RNA molecule forms a complex by binding to the RNA probe and thereby becomes capable of emitting fluorescence. On the other hand, in the absence of a target substance, the RNA molecule undergoes degradation by the exoribonuclease. In this case, neither can the RNA probe bind nor can the optical signal be generated because the fluorescent RNA aptamer sequence is also degraded.

In the present aspect, a plurality of different target substances may be detected using, at the same time, a plurality of RNA molecules differing in the sequence of the recognition region and the sequence of the reporter region. The present aspect can also be interpreted as an invention that relates to a composition or a kit for the detection of a target substance, comprising the RNA molecule or the DNA construct for use in the detection method. The composition may include exoribonuclease, an RNA probe, and optionally RppH.

The method for detecting a target substance according to the third aspect is capable of performing detection reaction in a wide range of sample solutions, and it can be used in the detection of various target substances, such as the detection of contaminants in industrial products. The method for detecting a target substance according to the third aspect is also capable of detecting an inhibitory substance of a molecule involved in translation, and also produces the advantage that the detection system can be constructed with fewer materials.

### EXAMPLES

Hereinafter, the present invention will be described in more detail with reference to Examples. The following Examples are not intended to limit the present invention.

### Experimental method (1)

### Plasmid construction

### Preparation of switch plasmid

pIRES2-EGFP was linearized by PCR amplification. A fragment of N79 connected to an aptamer was prepared by PCR amplification. KOD One PCR Master Mix -Blue- from Toyobo Co., Ltd. (Code No. KMM-201) was used in both the cases of PCR amplification. The linearized pIRES2-EGFP and each fragment were subjected to in-fusion reaction using In-Fusion HD Cloning Kit from Clontech Laboratories, Inc. (Takara code: Z9649N) to prepare a switch plasmid. The primer set shown in Table 1 was used in the preparation of the switch plasmid.

**Table 1**

| | Primer name | Sequence | Seq ID No. |
|---|---|---|---|
| 1 | pIRES2-EGFP_iPC R_Fw | TCGAGCTCAAGCTTCGAATTCTGC | 1 |
| 2 | pIRES2-EGFP_iPC R_Rv | GCTAGCGGATCTGACGGTTCAC | 2 |
| 3 | Rz_N79_code | CTGAGATGCAGGTACATCCAGCTGATGAGTCCCAAATAGGACGAAACGCG CTTCGGTGCGTCCTGGATTCCACTGCTATCCAC | 3 |
| 4 | InFusion CMV-Rz_ N79_Fw | GAACCGTCAGATCCGCTAGCCTGAGATGCAGGTACATCCAGCTG | 4 |
| 5 | InFusion Rz_N79-AsCas12a_crRNA_ Rv | AATTCGAAGCTTGAGCTCGACCGGTAGATCTatctacaagagtagaaattGTGG ATAGCAGTGGAATCCAGGAC | 5 |
| 6 | InFusion Rz_N79-SaCas9_sgRNA_Rv | AATTCGAAGCTTGAGCTCGACCGGTAGATCTatctcgccaacaagttgacgagat aaacacggcattttgccttgttttagtagattctgtttccagagtactaaaacGTGGATAGCAG TGGAATCCAGGAC | 6 |

### Preparation of trigger plasmid

The open reading frame (ORF) of a Cas protein used as a trigger was amplified by PCR. Subsequently, the amplified ORF was cleaved with appropriate restriction enzymes and then inserted downstream of CMV promoter of pcDNA3.1-myc-HisA. The primer set and the templated plasmids shown in Table 2 were used in the preparation of the trigger plasmid.

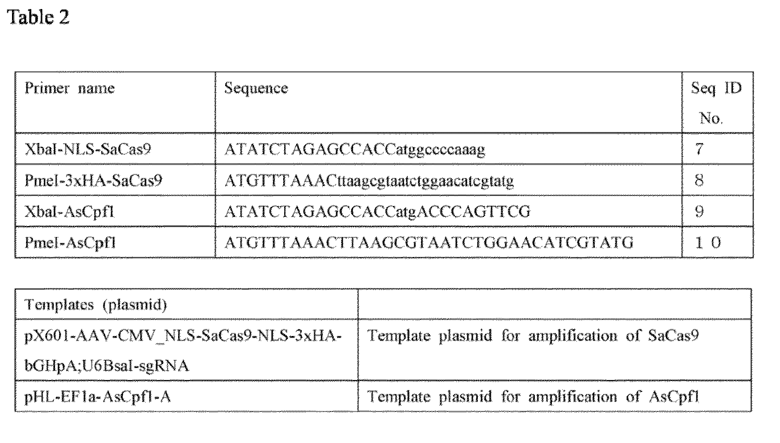

A plasmid for transfection of cultured cells was purified at a large scale using commercially available Midiprep kit (Qiagen N.V. or Promega Corp.).

### Construction of in vitro transcription (IVT) template

### Preparation of IVT template for switch

A templated structure for IVT template preparation for switch mRNA was amplified by PCR using the plasmid and the primer set shown in 7 to 9 of Table 3. An IVT template for a switch was amplified by PCR using this template and using the primer sets shown in Tables 4A and 4B.

**Table 3**

| | Primer/Oligo DNA name | Sequence | Seq ID No. |
|---|---|---|---|
| 1 | TAP_T7_G3C fwd primer | CAGTGAATTGTAATACGACTCACTATAGGGC | 1 1 |
| 2 | Cas protein O RF PCR | | |
| 3 | 3UTR120A | TTTTTTTTTTTTTTTTTTTTTTTTTTTTTTTTTTTTTTTTTTTTTTTTTTTTTT TTTTTTTTTTTTTTTTTTTTTTTTTTTTTTTTTTTTTTTTTTTTTTTTTTTTTT TTTTTTTTTTTTCCTACTCAGGCTTTATTCA | 1 2 |
| 4 | YF771_T7_5U TR_fwd | ATTGTAATACGACTCACTATAGGGCGAATTAAGAGAGAAAAGAAGAGTAAG | 1 3 |
| 5 | pUC19-iRFP67 0woT7 | | |
| 6 | 3UTR120A | TTTTTTTTTTTTTTTTTTTTTTTTTTTTTTTTTTTTTTTTTTTTTTTTTT TTTTTTTTTTTTTTTTTTTTTTTTTTTTTTTTTTTTTTTTTTTTTTTTTT TTTTTTTTTTTTCCTACTCAGGCTTTATTCA | 1 2 |
| 7 | 5UTR primer for CVB3_IRE S | GCGAATTAAGAGAGAAAAGAAGAGTAAGAAGAAATATAAGACACCGG | 1 4 |
| 8 | STKp-39 pUC 19 PIE-CVB3-EGFP | | |
| 9 | Rev3UTR2T20 | TTTTTTTTTTTTTTTTTTTTCCTACTCAGGCTTTATTCAAAGACCAAG | 1 5 |

**Table 4A**

| Primer name | Sequence | Seq ID No. |
|---|---|---|
| T7-GG-5UTR primer f or CVB3_IRES | CAGTGAATTGTAATACGACTCACTATAGGGCGAATTAAGAGAGAAAAGA AGAGTAAGAAGAAATATAAGACACCGG | 1 6 |
| T7-G+G+Gluc-Sa_gRN A | CAGTGAATTGTAATACGACTCACTATAGGAGATCAGGGCAAACAGAACgtt ttagtactctggaaacagaatctactaaaacaaggcaaaatgccgtgtttatctcgtcaacttgtt ggcgagat | 1 7 |
| Gluc-Sa_gRNA-5UTR p rimer for CVB3_IRES | ccgtgtttatctcgtcaacttgttggcgagatGCGAATTAAGAGAGAAAAGAAGAGTA AGAAGAAATATAAGACACCGG | 1 8 |
| T7-GG+Gluc-AsCas12a _crRNA-5UTR primer f or CVB3_IRES | CAGTGAATTGTAATACGACTCACTATAGGaatttctactcttgtagatAGATCAG GGCAAACAGAACTTTGAGCGAATTAAGAGAGAAAAGAAGAGTAAGAAGA AATATAAGACACCGG | 1 9 |
| T7-GG-5UTR-Gluc-AsC as12a_crRNA for CVB 3 IRES | CAGTGAATTGTAATACGACTCACTATAGGGCGAATTAAGAGAGAAAAGA AGAGTAAGAAGAAATATAAGACACCGGTcgccaccaatttctactcttgtagatAGA TCAGGGCAAACAGAACTTTGATTAAAACAGCCTGTGGGTTG | 2 0 |
| T7-G+Gluc-Sp_gRNA | CAGTGAATTGTAATACGACTCACTATAGGAGATCAGGGCAAACAGAACT GTTTTAGAGCTAGAAATAGCAagttaaaataaggctagtccgttatcaacttg | 2 1 |
| Gluc-Sp_gRNA-5UTR p rimer for CVB3_IRES | AGAAATAGCAagttaaaataaggctagtccgttatcaacttgaaaaagtggcaccgagtcg gtgcGCGAATTAAGAGAGAAAAGAAGAGTAAGAAGAAATATAAGACACCG GTcgccacc | 2 2 |
| T7-G+Gluc-Cj_gRNA | CAGTGAATTGTAATACGACTCACTATAGGAGATCAGGGCAAACAGAACgtt ttagtccctgaaaagggactaaaataaagagtttgcgggactctgcggggttacaatcccctaa aaccgc | 2 3 |
| Gluc-Cj_gRNA-5UTR p rimer for CVB3_IRES | gcggggttacaatcccctaaaaccgcGCGAATTAAGAGAGAAAAGAAGAGTAAGA AGAAATATAAGACACCGGTcgccacc | 2 4 |
| T7-GG+7cx4 Fw | GGtctagaAACTCTcGCCTATAATCGCGTGGATATGGCACGCAAGTTTCTA CCGGGCACCGTAAATGTCCGACTAGGCTAGAAGGTCCCAAGCCCTTATA AAGCAGAGGGAAAGATATCACTTTTAATGCTGCCTAGCtccgga | 2 5 |
| T7-7cx4 Fw | tctagaAACTCTcGCCTATAATCGCGTGGATATGGCACGCAAGTTTCTACC GGGCACCGTAAATGTCCGACTAGGCTAGAAGGTCCCAAGCCCTTATAAA GCAGAGGGAAAGATATCACTTTTAATGCTGCCTAGCtccgga | 2 6 |

**Table 4B**

| | | |
|---|---|---|
| 7cx4_v1 Fw | GGtctagaAACTCTcGCCTATAATCGCGTGGATATGGCACGCAAGTTTCTA CCGGGCACCGTAAATGTCCGACTAGGCTAGAAGTGCCCAAGCCCTTATA AAGCAGAGGGAAAGATATCACTTTTAATGCTGCCTAGCtccgga | 2 7 |
| 7cx4_v2 Fw | GGtagaAACTCTcGCCTATAATCGCGTGGATATGGCACGCAAGTTTCTACC GGGCACCGTAAATGTCCGACTAGGCTACCAGTGCCCAAGCCCTTATAAA GCAGAGGGAAAGATATCACTTTTAATGCTGCCTAGCtccgga | 2 8 |
| 7cx4_v3 Fw | GGtctagaAACTCTcGCCTATAATCGCGTGGATATGGCACGCAAGTTTCTA CCGGGCACCGTAAATGTCCGACTAGGCTAGAAGGTCCCAAGCCCTTGCC TAGCtccgga | 2 9 |
| 7cx4_v4 Fw | GGtagaAACTCTcGCCTATAATCGCGTGGATATGGCACGCAAGTTTCTACC GGGCACCGTAAATGTCCGACTAGGCTACCAGGTCCCAAGCCCTTGCCTA GCtccgga | 3 0 |
| T7-G-HCV_1a-CVB3_I RES-EGFP_Fw | CAGTGAATTGTAATACGACTCACTATAGgccagccccctgatgggggcgacactcc accatgaatcactcccctgGCGAATTAAGAGAGAAAAGAAGAGTAAGAAGAAAT ATAAGACACCGG | 3 1 |
| T7-GG-HCV_2a-CVB3_ IRES-EGFP_Fw | CAGTGAATTGTAATACGACTCACTATAGGacctgcccctaataggggcgacactcc gccatgaatcactcccGCGAATTAAGAGAGAAAAGAAGAGTAAGAAGAAATAT AAGACACCGG | 3 2 |
| 3'UTR-120A primer | TTTTTTTTTTTTTTTTTTTTTTTTTTTTTTTTTTTTTTTTTTTTTTTTTTTTTTTTTTTT TTTTTTTTTTTTTTTTTTTTTTTTTTTTTTTTTTTTTTTTTTTTTTTTTTTTTTTTTTTT TTTTTTTTTTTTTTTTTCCTACTCAGGCTTTATTCA | 1 2 |

### Preparation for IVT template for trigger

The ORF of a protein used as a trigger, and 5'-UTR and 3'-UTR sequences were amplified by PCR from a plasmid or oligo DNA using appropriate primers. The templated plasmid for ORF preparation and the primers used are as described in Table 5, and the templated oligo DNA for 5'-UTR and 3'-UTR preparation and the primers used are as described in Table 6. Then, in order to prepare an IVT template for trigger mRNA synthesis, the 5'-UTR fragment, the 3'-UTR fragment, and the ORF were linked by PCR amplification using the primer set shown in 1 to 3 of Table 3.

**Table 5**

| Primer/Oligo DNA name | Sequence | Seq I D No. |
|---|---|---|
| AsCas12a ORF_Fw | CACCGGTCGCCACCATGACCCAGTTCGAGGGCTTCACTAATC TG | 3 3 |
| pcDNA3.1-AsCpf1 | | |
| AsCas12a ORF_Rv | GCCCCGCAGAAGGTCTAGACTAAGCGTAATCTGGAACATCG TATGGGTAAGC | 3 4 |
| SaCas9 ORF_Fw | CACCGGTCGCCACCATGgccccaaagaagaagcggaagg | 3 5 |
| pcDNA3.1-SaCas9 | | |
| SaCas9 ORF_Rv | GCCCCGCAGAAGGTCTAGACTAagcgtaatctggaacatcgtatggg taagcg | 3 6 |
| CjCas9 ORF_Fw | CACCGGTCGCCACCATGgccagaatcctggc | 3 7 |
| pcDNA3.1-CjCas9 | | |
| CjCas9 ORF_Rv | GCCCCGCAGAAGGTCTAGACTAggcgtagtcgggcacgtcgtag | 3 8 |
| SphcCas9 ORF fwd primer | CACCGGTCGCCACCATGGATAAGAAATACAGCATTGGAC | 3 9 |
| pHL-EF1a-SphcCas9-iC-A | | |
| SphcCas9 ORF rev primer | GCCCCGCAGAAGGTCTAGACTATCACACCTTCCTCTTCTTCT TGG | 4 0 |

**Table 6**

| No. | Primer/Oligo DNA name | Sequence(5' -> 3') | Seq ID No. |
|---|---|---|---|
| 1 | TAP_T7_G3C fwd primer | CAGTGAATTGTAATACGACTCACTATAGGGC | 4 1 |
| 2 | IVT_5prime_ UTR primer | CAGTGAATTGTAATACGACTCACTATAGGGCGAATTAAGAGAGA AAAGAAGAGTAAGAAGAAATATAAGACACCGGTCGCCACCATG | 4 2 |
| 3 | Rev5UTR pri mer | CATGGTGGCGACCGGTGTCTTATATTTCTTCTTACTC | 4 3 |
| 4 | IVT_3prime_ UTR primer | TCTAGACCTTCTGCGGGGCTTGCCTTCTGGCCATGCCCTTCTTCT CTCCCTTGCACCTGTACCTCTTGGTCTTTGAATAAAGCCTGAGTA GG | 4 4 |
| 5 | Fwd3UTR pri mer | TCTAGACCTTCTGCGGGGC | 4 5 |
| 6 | Rev3UTR2T20 | TTTTTTTTTTTTTTTTTTTTTTTTCCTACTCAGGCTTTATTCAAAGACCA AG | 1 5 |

### Preparation of IVT template for reference

An IVT template for reference mRNA was prepared by PCR amplification using the plasmid and the primer set shown in 4 to 6 of Table 3.

The PCR product was purified using Monarch PCR & DNA Cleanup Kit from New England Biolabs Inc. (#T1030) in accordance with the manual attached to the kit. The product amplified by PCR with the plasmid as a template was incubated at 37°C for 30 minutes using Dpn I from Toyobo Co., Ltd. (Code No. DPN-101) to digest the plasmid.

### Synthesis and purification of mRNA

mRNA was synthesized using MEGAscript T7 Transcription Kit from Thermo Fisher Scientific Inc. (Catalog No. AMB13345). In this reaction, 1-methylpseudouridine-5'-triphosphate (TriLink BioTechnologies) was used instead of each uridine triphosphate, in addition to switch mRNA. The guanosine triphosphate used was diluted 5-fold with ARCA (Anti-Reverse Cap Analog) from New England Biolabs Inc. or TriLink BioTechnologies (Catalog # S1411 or N-7003-10). Another switch mRNA was prepared so as to have an A-Cap structure having no translational activity using guanosine triphosphate diluted 5-fold with G(5')ppp(5')A RNA Cap Structure Analog from New England Biolabs Inc. (Catalog # S1406). The reaction mixed solution was reacted at 37°C for 4 to 6 hours. Then, TURBO DNase from Thermo Fisher Scientific Inc. (Catalog No. AM2238) was added thereto, and the mixture was further isothermally treated at 37°C for 30 minutes. The obtained mRNA was purified using Monarch RNA Cleanup Kit from New England Biolabs Inc. (Catalog # T2040). The mRNA thus purified was 5'-terminally dephosphorylated, and the resulting sample was isothermally treated at 37°C for 30 minutes using Antarctic Phosphatase (Catalog # M0289) and Antarctic Phosphatase Reaction Buffer (Catalog # B0289) from New England Biolabs Inc. Then, the sample was purified using Monarch RNA Cleanup Kit (New England Biolabs Inc.). mRNA having a monophosphate group at the 5' end was prepared by transcription using guanosine triphosphate diluted 5-fold with GMP-Solid (Jena Bioscience GmbH; NU-1028-5G) or by isothermal treatment at 37°C for 30 minutes using RNA 5' Pyrophosphohydrolase from New England Biolabs Inc. (Catalog # M0356).

### Cell culture

HEK293FT cells were cultured under conditions of 37°C and 5% CO₂ using DMEM (Nacalai Tesque, Inc.) supplemented with 10% FBS, 2 mM L-Glutamine from Thermo Fisher Scientific Inc. (Catalog No. 25030149), 0.1 mM Non-Essential Amino Acids Solution from Thermo Fisher Scientific Inc. (Catalog No. 11140050), and 1 mM Sodium Pyruvate (Sigma).

### Transfection with plasmid

293FT cells were placed in a 24-well plate 24 hours before transfection. Plasmids were introduced thereto using Lipofectamine 2000 Transfection Reagent from Thermo Fisher Scientific Inc. (Catalog No. 11668019) in accordance with the manual attached thereto. 100 ng of the switch plasmid, 400 ng of the trigger plasmid, and 100 ng of the reference plasmid were mixed in Opti-MEM from Thermo Fisher Scientific Inc. (Catalog No. 51985091) and used in co-transfection. The reference plasmid used was an iRFP670 expression plasmid and used as a transfection control.

### Transfection with mRNA

293FT cells were placed in a 24-well plate 24 hours before transfection. mRNA was introduced thereto using 1 µL of Lipofectamine messenger MAX Transfection Reagent from Thermo Fisher Scientific Inc. (Catalog No. LMRNA015) in accordance with the manual attached thereto. 100 ng of the switch mRNA, 200 ng of the trigger mRNA, and 100 ng of the reference mRNA were mixed in Opti-MEM and used in the co-transfection of the 293FT cells. The reference mRNA used was iRFP670 expression mRNA and used as a transfection control.

### XRN knockdown and transfection with mRNA

293FT cells were placed in a 6-well plate 24 hours before transfection with small interfering RNA (siRNA). 25 pmol of siRNA (12.5 pmol each under a condition of siXRN1 + siXRN2) was introduced into the cells using 7.5 µL of Lipofectamine RNAi MAX Transfection Reagent from Thermo Fisher Scientific Inc. (Catalog No. 13778150) in accordance with the manual attached thereto. The 293FT cells were placed in a 24-well plate 48 hours after siRNA introduction. After 24 hours, siRNA and mRNA were introduced thereto using 1 µL of Lipofectamine Messenger MAX Transfection Reagent in accordance with the manual attached thereto. 2.5 pmol of the siRNA (1.25 pmol each under a condition of siXRN 1 + siXRN2), 100 ng of the switch mRNA, and 100 ng of the reference mRNA were mixed in Opti-MEM and used in co-transfection. The reference mRNA used was iRFP670 expression mRNA and used as a transfection control.

### XRN knockdown and transfection with plasmid

293FT cells were placed in a 6-well plate 24 hours before transfection with small interfering RNA (siRNA). 25 pmol of siRNA (12.5 pmol each under a condition of siXRN1 + siXRN2) was introduced thereto using 7.5 µL of Lipofectamine RNAi MAX Transfection Reagent in accordance with the manual attached thereto. The 293FT cells were placed in a 24-well plate 48 hours after siRNA introduction. After 24 hours, siRNA and plasmids were introduced thereto using Lipofectamine 2000 Transfection Reagent in accordance with the manual attached thereto. 2.5 pmol of the siRNA (1.25 pmol each under a condition of siXRN1 + siXRN2), 100 ng of the switch plasmid, and 100 ng of the reference plasmid were mixed in Opti-MEM and used in co-transfection. The reference plasmid used was an iRFP670 expression plasmid and was used as a transfection control.

### Cell imaging

Fluorescence micrographs were taken using Cytell Cell Imaging System (GE Healthcare Life Sciences). The brightness and contrast of the fluorescence micrographs were edited using ImageJ software (NIH).

### Flow cytometry

On the day following transfection, the cells were separated from the plate, passed through a mesh, and analyzed by flow cytometry. Accuri C6 (BD Biosciences) was used in the experiment using the 24-well plate. EGFP was detected using FL1 (530/30 nm) filters. iRFP670 was detected using FL4 (675/12.5 nm) filters. Dead cells and debris were excluded by forward and side scattered light signals. In the calculation of translation efficiency using plasmids, live cells having a fluorescence value of iRFP670 beyond or at a given level were used as cells of interest in analysis.

### Results

### Design of ON switch

A typical mRNA has a cap structure at the 5' end followed by connected sequences such as a 5' untranslated region (5'-UTR), a gene of interest (ORF), and a 3' untranslated region (3'-UTR) with a poly(A) tail (FIG. 1(a)). Since the ON switch has no cap structure at the 5' end, its resistance to degradation by exoribonuclease from the 5' end is weakened. In a typical mRNA, the 5'-terminal cap structure is essential for the initiation of translation. Therefore, an ON switch lacking this cap structure requires another method for the initiation of translation. Hence, for the ON switch, an internal ribosome entry site (IRES) was inserted upstream of the gene of interest in order to cause translation. Ligand-dependent translation can be caused by inserting an RNA sequence (recognition region) having the ability to bind to a particular target substance (ligand) to 5'-UTR upstream of the IRES (FIG. 1(b)). In short, the ON switch has no cap structure and is therefore degraded by intracellular exoribonuclease in the absence of the ligand (OFF state), whereas the degradation of the ON switch is presumably suppressed by the specific binding of the recognition region embedded in the artificial mRNA to the ligand in the presence of the ligand (ON state) so that the freely selected protein of interest is translated (FIG. 1(c)). In this driving principle, it is considered that various types of molecules can be detected because the function of the switch does not depend on the type of aptamer.

### Design of plasmid-based mRNA ON switch

A plasmid for intracellular expression of the artificial mRNA designed as mentioned above was constructed. First, an RNA sequence (aptamer) capable of binding to a target substance was inserted to the 3' side of N79 (a modified form of *Schistosoma mansoni-*derived ribozyme Sml), a type of self-cleaving ribozyme, and then, IRES and a freely selected open reading frame (ORF) were placed to construct a plasmid-based mRNA ON switch. Here, SaCas9 or AsCpfl was used as the target substance (ligand). The switch was designed using the sequence of known crRNA (CRISPR RNA) or sgRNA (single guide RNA; chimeric RNA of crRNA and trans-activating crRNA (tracrRNA)) binding to each Cas protein as the aptamer. In transcribed mRNA, self-cleavage is first caused by N79 to give rise to mRNA having no cap structure (FIG. 2A). This mRNA is intracellularly degraded because of having no cap structure, and as a result, its translation is repressed (FIG. 2B, upper). On the other hand, by introducing each Cas protein, the degradation of the switch by exoribonuclease is presumably suppressed because the Cas protein specifically recognizes and binds to crRNA/sgRNA embedded in the mRNA (FIG. 2B, lower).

### Functional evaluation of Cas protein-responsive plasmid ON switch

A plasmid for the expression of the ON switch (switch plasmid) was prepared on the basis of the design mentioned above, and was introduced into 293FT cells, together with a plasmid for the expression of the Cas protein (trigger plasmid) and a transfection control plasmid for the expression of iRFP670 (reference plasmid for the expression of the protein irrespective of the presence of the target substance). FIG. 3 is a graph showing the translation efficiency of each switch plasmid. The translation efficiency was determined by first dividing the fluorescence intensity of GFP by the fluorescence intensity of iRFP670 as to each switch, and subsequently dividing the value at the time of trigger introduction by the value without trigger introduction. Comparison was further made with reference to a control switch plasmid. The error bar depicts mean ± standard deviation (n = 3, three independent experiments). The translation efficiency of the switch using AsCpfl (FIG. 3(a)) and SaCas9 (FIG. 3(b)) was 1.9 times and 7.8 times, respectively. Thus, the ON switch having this structure was confirmed to function by DNA introduction.

### Functional evaluation of Cas protein-responsive mRNA ON switch

The ON switch was then tested to see if it worked when introducing an in vitro transcribed mRNA molecule directly into cells. A mRNA ON switch (switch mRNA) was prepared by *in vitro* transcription and introduced to 293FT cells, together with mRNA for the expression of the Cas protein (trigger mRNA) and transfection control mRNA for the expression of iRFP670 (reference mRNA). Here, SaCas9, CjCas9, SpCas9, or AsCpfl was used as the target substance (ligand). FIG. 4 is a graph showing the translation efficiency of each switch mRNA using SaCas9 (a), CjCas9 (b), SpCas9 (c), or AsCpfl (d) as the target substance. The translation efficiency was determined by first dividing the fluorescence intensity of GFP by the fluorescence intensity of iRFP670 as to each switch, and subsequently dividing the value at the time of trigger introduction by the value without trigger introduction. Comparison was further made with reference to control switch mRNA. The error bar depicts mean ± standard deviation (n = 3, three independent experiments). High translation efficiency was obtained in 3 except for AsCpfl out of the four switch mRNAs.

### Functional improvement of mRNA ON switch by change in insertion position of aptamer

Functional improvement in switch was attempted, as to the AsCpfl-responsive switch having low translation efficiency among the Cas protein-responsive mRNA ON switches described above, by moving the insertion position of the aptamer to the 3' side (FIG. 5). GG+Gluc-AsCas12a_crRNA-CVB3_IRES-EGFP of FIG. 5(a) is the AsCpf1-responsive switch used in FIG. 4(d). Based on this, it was confirmed that the translation efficiency of the switch is improved by changing the number of bases residing on the 5' side of the aptamer (FIG. 6). Thus, each Cas protein was successfully detected through the use of crRNA/gRNA appropriate for the Cas protein. These results suggest that the amplitude of detection sensitivity of the mRNA ON switch can be adjusted by applying this. Glue was used as a sequence considered to have small influence on the switch or an intracellular molecule, as a portion of the crRNA sequence. CVB3 refers to coxsackievirus B group type 3-derived

### IRES.

Suppression of degradation of mRNA ON switch in the OFF state by knockdown of XRN XRN, a type of exoribonuclease, degrades intracellular RNA in the 5' → 3' direction, and works to maintain the level of RNA turnover. XRN includes two types: XRN1 present in the cytoplasm and XRN2 present in the nucleus. XRN1, in particular, is known to degrade mRNA from which the 5'-terminal cap structure has been removed for mRNA metabolism. Accordingly, it was hypothesized that these XRN proteins would contribute to intracellular degradation of the mRNA ON switch in an OFF state. In order to test this hypothesis, each XRN was knocked down, and a control mRNA ON switch was introduced. The control mRNA ON switch refers to a mRNA switch that includes no recognition region and does not contain a 5' cap structure. The control mRNA ON switch harboring an *in vitro* synthesized messenger was translationally promoted in an OFF state by only the knockdown of XRN1 (FIG. 7(a)), and the plasmid-based mRNA ON switch (SaCas9-responsive switch used in FIG. 3(b)) was translationally promoted in an OFF state only under conditions involving XRN1 and XRN2 both knocked down (FIG. 7(b)). The error bar depicts mean ± standard deviation (n = 3, three independent experiments). It is considered that the ON switch introduced through plasmid DNA is transcribed in the nucleus and then transported to the cytoplasm. Therefore, the results obtained this time support the hypothesis that XRN2 and XRN1 contribute to the degradation of the mRNA ON switch in the nucleus and in the cytoplasm, respectively.

### Preparation of small molecule-responsive mRNA ON switch

In principle, the present ON switch is driven by target substance (ligand)-dependent inhibition of degradation by exoribonuclease from the 5' end. It is considered that such inhibition of degradation can be achieved not only by the binding of a protein, but also by the binding of a target substance leading to an RNA structure having degradation resistance at the 5' end. In order to test this idea, a guanine-responsive ON switch was designed. For guanine detection, a mRNA ON switch was prepared using known guanine-responsive aptazyme. This aptazyme is structurally changed in the presence of guanine so that self-cleavage by the active site of the ribozyme is suppressed. This structural change is considered to induce a 5'-terminal recess. The activity of degrading 5'-terminally recessed RNA (RNA protected by double-strand binding) is known to be low as a feature of XRN. Accordingly, the present inventors believed that an ON switch could be prepared by adding this aptazyme in which self-cleaving ability was deactivated to the 5' end and thereby inducing a guanine-dependent 5'-terminal recess (FIG. 8A). The ON switch was prepared by introducing a mutation so as to deactivate the known guanine-responsive aptazyme (v1), designing the switch so as to more reduce terminal exposure (v2), deleting a portion of the ribozyme moiety such that the aptazyme activity disappeared (v3), and designing v3 so as to further reduce terminal exposure (v4). As a result of introducing the prepared guanine-responsive ON switch to cells cultured in a medium supplemented with guanine, guanine-dependent translational activity was exhibited. GG+7c4x_v3-CVB3_IRES-EGFP, in particular, functioned as the most efficient ON switch (FIG. 8B). The error bar depicts mean ± standard deviation (n = 3, three independent experiments). Accordingly, the ON switch according to the present invention can also be used in the detection of a low-molecular compound.

### Preparation of miRNA-responsive mRNA ON switch

For the purpose of detecting miR-122, a type of micro-RNA (miRNA), for hepatitis C virus (HCV), which is an RNA virus causing hepatitis C, a mRNA ON switch was prepared by incorporating the 5'-terminal structure of HCV RNA to the recognition region. The miRNA or a RISC complex binds to the miR-122-responsive sequence and thereby escapes degradation by XRN (FIG. 9(a)). As a result of using the sequence of HCV RNA of genotype 1a or 2a, the promotion of translation was able to be confirmed in the presence of the RISC complex serving as a trigger (2.3 times and 1.4 times for the genotypes 1a and 2a, respectively). The error bar depicts mean ± standard deviation (n = 3, three independent experiments) (FIG. 9(b)). These results suggested that intracellular miRNA can also be detected using the ON switch according to the present invention.

### Promotion of degradation of mRNA ON switch by 5-terminal monophosphorylation

XRN is known to degrade RNA having monophosphate at the 5' end. The end of the mRNA ON switch prepared by *in vitro* transcription this time was considered to have a triphosphate group or a hydroxyl group. From the results described above, the introduced mRNA ON switch is presumably intracellularly monophosphorylated and then degraded by XRN. In the case of introducing a mRNA ON switch directly to cells, the expression of a reporter can be slightly observed even in an OFF state (leaky expression). This is considered to be partly because translation occurs before the mRNA ON switch, after being introduced to cells, is degraded through the steps mentioned above. Accordingly, the present inventors believed that an expression level in an OFF state (leaky expression) could be further decreased by introducing a mRNA ON switch in a state in which the 5' end is monophosphorylated. A mRNA ON switch was prepared by performing dephosphorylation treatment with RppH after transcription or monophosphorylating the 5' end using GMP at the time of transcription. The introduction of the resulting mRNA ON switch to cells successfully reduced a fluorescence intensity in an OFF state of the monophosphorylated mRNA ON switch as compared with a sample without 5'-terminal treatment (FIG. 10).

### A. Study on ribozyme for cap removal at time of plasmid introduction

### Experimental method

### Switch plasmid construction

A plasmid was prepared with the prepared N79-SaCas9 aptamer-containing plasmid as a template using the primer set shown in Table 7 and Q5 Site-Directed Mutagenesis Kit (New England Biolabs Inc.; E0554S). A plasmid for transfection of cultured cells was purified at a large scale using commercially available Midiprep kit (Qiagen N.V or Promega Corp.).

**Table 7**

| Primer name | Sequence | Seq ID No. |
|---|---|---|
| KWC993_pHHR(6bp)-Sa_gRNA_Rv | gacgagcttactcgtttcgtcctcacggactcatcaggttttacGCTAGCGG ATCTGACGGTTCAC | 46 |
| KWC994_pHHR(7bp)-Sa_gRNA_Rv | gacgagcttactcgtttcgtcctcacggactcatcaggttttaGCTAGCGGA TCTGACGGTTCAC | 47 |
| KWC995_Sa_gRNA_Fw | gttttagtactctggaaacagaatctactaaaacaag | 48 |

### Transfection

Transfection was performed in the same manner as described in the preceding experimental method.

### Cell imaging

Fluorescence micrographs were taken using CQ1 confocal image cytometer (Yokogawa Electric Corporation). The brightness and contrast of the fluorescence micrographs were edited using ImageJ software (NIH).

### Flow cytometry

Flow cytometry was performed in the same manner as described in the preceding experimental method.

### Results

For a plasmid-based mRNA ON switch, the cap structure was removed from transcribed mRNA through the use of self-cleaving ribozyme N79. However, the self-cleavage by N79 allows a portion of the N79 to remain in a newly formed 5' region. FIG. 2A schematically illustrates a structural prediction diagram of N79 composed of 83 bases, and the structures of a 5'-terminal fragment (bases 1 to 62) and a 3'-terminal fragment (bases 63 to 83) after self-cleavage indicated by the arrow. In the case of designing an RNA molecule containing N79 as a 5' cap structure-removing sequence on the 5' side of the recognition region, the bases at positions 63 to 83 of N79 are toward the 5' end of mRNA after cap removal, by self-cleavage. Hence, limitations may occur on a sequence subsequent to the recognition region such as an aptamer inserted downstream of N79. Accordingly, this problem can be solved, provided that a sequence proximal to the 5' end of mRNA after cap removal can be freely designated. For this purpose, hammerhead ribozyme (HHR) which is a modified form of tobacco ringspot virus-derived ribozyme was used. FIG. 11 illustrates a structural prediction diagram of HHR and its self-cleavage site indicated by the arrow. HHR is known to exhibit self-cleaving activity through a stem structure (double-stranded RNA region) consisting of freely selected 6 base pairs (represented by N). The RNA sequence (5' nnnnnncugaugaguccgugaggacgaaacgaguaagcucgucnnnnnn 3') of the HHR shown in FIG. 11 is shown in SEQ ID NO: 49.

In the present experiment, a SaCas9-responsive mRNA switch containing HHR, the sequence of which was designed so as to form 6 base pairs as usual (HHR (6 bp)) or HHR elongated to 7 base pairs (7 bp), was designed and evaluated for its performance. The evaluation results are shown in FIGs. 12A and 12B. The Rz_N79 represents a SaCas9-responsive switch using N79, the Positive represents a reporter plasmid for expression of EGFP from IRES without a cap removal mechanism, and the Cap-dependent represents a reporter plasmid for expression of EGFP via a 5' cap, as in typical mRNA. FIG. 12A is a diagram showing, in dot plots and histograms, change in cell population under each condition. Use of Rz_N79 was found to exhibit almost the same EGFP expression as that by single introduction of iRFP670 in Trigger- (without trigger introduction; OFF state). In the case of using HHR, HHR with a base pair length of 7 bp was found to reduce an expression level in an OFF state. FIG. 12B shows a median value of EGFP/iRFP670 brightness in each construct. Rz_N79 exhibited the best ON/OFF ratio. On the other hand, the system based on HHR was shown to be able to activate SaCas9-dependent translation of the reporter as intended, though the ON/OFF ratio was poorer.

### B. Test on dependence of ON switch on amount of trigger

### Experimental method

### Transfection

293FT cells were placed in a 24-well plate 24 hours before transfection. The mRNA was introduced thereto using 1 µL of Lipofectamine messenger MAX Transfection Reagent from Thermo Fisher Scientific Inc. (Catalog No. LMRNA015) in accordance with the manual attached thereto. Then, 100 ng of the switch mRNA, 0 to 200 ng of the trigger mRNA (corrected with control mRNA so as to attain a total of 200 ng), and 100 ng of the reference mRNA were mixed in Opti-MEM and used in the co-transfection of the 293FT cells. The control mRNA used was hRluc expression mRNA, and the reference mRNA used was iRFP670 expression mRNA and used as a transfection control.

### Cell imaging

Cell imaging was performed in the same manner as described in the experimental method of A.

### Flow cytometry

Flow cytometry was performed in the same manner as described in the preceding experimental method.

### Results

An ON switch was tested for whether it exhibited reporter activity in a manner dependent on the amount of a trigger. A SaCas9-responsive mRNA ON switch (switch mRNA) was prepared by *in vitro* transcription and introduced to 293FT cells, together with mRNA for the expression of the SaCas protein in various amounts (trigger mRNA) and transfection control mRNA for the expression of iRFP670 (reference mRNA). FIG. 13 is a graph showing the translational activation efficiency of each trigger mRNA when an ON/OFF reporter expression level without trigger mRNA introduction is defined as 1. The control mRNA ON switch refers to a mRNA switch that includes no recognition region and does not contain a 5' cap structure. Also, each mRNA having an A-Cap structure and having resistance to degradation by exoribonuclease was provided. The introduced control mRNA or the introduced mRNA having an A-Cap structure was not translationally activated, irrespective of the amount of the trigger and the presence or absence thereof, whereas the SaCas9-responsive mRNA ON switch was shown to cause translational activation of the reporter in a manner dependent on the amount of the trigger.

### C. In vitro detection of target molecule

### Experimental method

### Preparation of IVT template

The sequence of an IVT template of Sa_gRNA-3WJdB, Sp_gRNA-3WJdB was constructed by PCR using the synthetic oligo DNA set shown in Table 8.

**Table 8**

| name | Sequence | Seq ID No. |
|---|---|---|
| KWC949_Sa _gRNA_anne al | CAGTGAATTGTAATACGACTCACTATAGGAGATCAGGGCAA ACAGAACgttttagtactctggaaacagaatctactaaaacaaggcaaaatgccgtgtttatct cgtcaacttgttggcgagatGCGAATTAAGAGAGAAAAGAAGAGTAAGA AGAAAT | 50 |
| KWC950_Sp _gRNA_anne al | CAGTGAATTGTAATACGACTCACTATAGGAGATCAGGGCAA ACAGAACTGTTTTAGAGCTAGAAATAGCAagttaaaataaggctagtcc gttatcaacttgaaaaagtggcaccgagtcggtgcGCGAATTAAGAGAGAAAAG AAGAGTAAGAAGAAAT | 51 |
| KWC958_Sp accr for 3WJdB attachment | GCGAATTAAGAGAGAAAAGAAGAGTAAGAAGAAATCCCACA TACTCTGATGATCC | 52 |
| KWC959_Rc v primer for 3WJdB attachemnt | CCCACATACACATGGCAAGAGCCCACACTCTACTCGACAGA TACGAATATCTGGACCCGACCGTCTCTTGCCATGAATGATC CGAGCCCACACTCTACTCGACAGATACGAATATCTGGACCC GACCGTCTCGGATCATCAGAGTATGTGGGATTTCTTC | 53 |

### Synthesis and purification of RNA

RNA was synthesized using MEGAshortscript T7 Transcription Kit from Thermo Fisher Scientific Inc. (Catalog No. AM1354). The reaction mixed solution was reacted at 37°C for 4 to 6 hours. Then, TURBO DNase from Thermo Fisher Scientific Inc. (Catalog No. AM2238) was added thereto, and the mixture was further isothermally treated at 37°C for 30 minutes to 1 hour. The obtained RNA was purified using Monarch RNA Cleanup Kit from New England Biolabs Inc. (Catalog # T2040). An aliquot of the purified product was further electrophoresed using a polyacrylamide gel containing 8 M urea (denatured PAGE), and the RNA product of interest was excised and isothermally treated overnight with HSC Buffer (400 mM NaCl, 25 mM Tris-HCl (pH 7.6), 0.1% SDS). Then, RNA was purified from the RNA eluate using phenol:chloroform:isoamyl alcohol = 25:24:1 (Nacalai Tesque, Inc.; 25970-14). For the synthesis of SaCas_gRNA, SpCas_gRNA, the oligos of Table 9 were added in equal amounts and used as a template.

**Table 9**

| name | Sequence | Seq ID No. |
|---|---|---|
| KWC937_ Sa_gRNA _temp | ATTTCTTCTTACTCTTCTTTTCTCTCTTAATTCGCatctcgccaacaagttgacgagataa acacggcattttgccttgttttagtagattctgtttccagagtactaaaacGTTCTGTTTGCCCTGA TCTCCTATAGTGAGTCGTATTACAATTCACTG | 54 |
| KWC938_ Sp_gRNA _temp | ATTTCTTCTTACTCTTCTTTTCTCTCTTAATTCGCgcaccgactcggtgccactttttcaagt tgataacggactagccttattttaactTGCTATTTCTAGCTCTAAAACAGTTCTGTTTGCCC TGATCTCCTATAGTGAGTCGTATTACAATTCACTG | 55 |
| KWC949_ Sa_gRNA _anneal | CAGTGAATTGTAATACGACTCACTATAGGAGATCAGGGCAAACAGAACgttttagta ctctggaaacagaatctactaaaacaaggcaaaatgccgtgtttatctcgtcaacttgttggcgagatG CGAATTAAGAGAGAAAAGAAGAGTAAGAAGAAAT | 56 |
| KWC950_ Sp_gRNA _anneal | CAGTGAATTGTAATACGACTCACTATAGGAGATCAGGGCAAACAGAACTGTTTT AGAGCTAGAAATAGCAagttaaaataaggctagtccgttatcaacttgaaaaagtggcaccgag tcggtgcGCGAATTAAGAGAGAAAAGAAGAGTAAGAAGAAAT | 57 |

### In vitro target molecule-dependent inhibition of RNA degradation

240 nM RNA was mixed with 2.5 units of XRN1 (New England Biolabs Inc.; M0338S), 20 units of RNase Inhibitor, Murine (New England Biolabs Inc.; M0314), 10 units of RppH (New England Biolabs Inc.; M0356S), and 200 nM EnGen Sau Cas9 (NEB; M0654T) or EnGen Spy Cas9 NLS (NEB; M0646M) (already diluted into 1 µM using Diluent B (NEB; B8002S)) so as to attain a total of 20 µL using ultrapure water in 1 x NEBuffer 3, isothermally treated at 37°C for 1 hour, and then treated at 70°C for 20 minutes. 5 µL of the reaction solution was mixed with the same amount as the amount of the RNA Loading Dye (2X) (New England Biolabs Inc.; B0363), treated at 95°C for 5 minutes, and then electrophoresed using SuperSep RNA, 15%, 17-well (FUJIFILM Wako Pure Chemical Corporation; 194-15881). The gel after electrophoresis was stained with SYBR Green II Nucleic Acid Gel Stain (Takara Bio Inc.; 5770A) and was photographed using Typhoon FLA 7000 (GE Healthcare).

### In vitro detection of target molecule using fluorescent aptamer

250 nM RNA was mixed with 2.5 units of XRN1 (New England Biolabs Inc.; M0338S), 20 units of RNase Inhibitor, Murine (New England Biolabs Inc.; M0314), 10 units of RppH (New England Biolabs Inc.; M0356S), 200 nM EnGen Sau Cas9 (NEB; M0654T) or EnGen Spy Cas9 NLS (NEB; M0646M) (already diluted into 1 µM using Diluent B (NEB; B8002S)), and 0.1 mM DFHBI-1T (Sigma; SML2697-5MG) so as to attain a total of 20 µL using ultrapure water in 1 x NEBuffer 3. The reaction solution thus prepared was immediately fluorescently measured in QuantStudio3 (Thermo Fisher Scientific Inc.). The fluorescent measurement was performed 24 times every 5 minutes. Then, the reaction solution was treated at 70°C for 20 minutes and treated at 4°C.

### Results

For *in vitro* reconstitution of the present ON switch mechanism, whether degradation by XRN1 could be inhibited *in vitro* in a manner dependent on a target substance was tested. SaCas_gRNA and SpCas_gRNA were synthesized *in vitro* as RNAs binding to SaCas9 and SpCas9, respectively, and examined for whether RNA degradation was regulated in response to a state in which the monophosphorylating enzyme RppH and the SaCas9 or SpCas9 protein were present in a state supplemented with XRN1. In the absence of RppH, it is expected that RNA would neither be monophosphorylated nor would undergo degradation by XRN1. In the presence of RppH, it is expected that RNA degradation is inhibited, provided that each binding Cas9 protein is present. FIG. 14 shows RNA degradation confirmed by gel electrophoresis after reaction under each condition of SaCas_gRNA or SpCas_gRNA. As shown therein, the degradation of each RNA was inhibited only in the absence of RppH or when the binding Cas9 protein was added (arrow) and occurred in the presence of RppH (XRN1 activity).

Next, whether the presence or absence of a target substance in a reaction solution could be determined using the present ON switch mechanism was tested. For this purpose, a complexed RNA molecule was prepared which consisted of a complex of an aptamer (recognition region) and a fluorescent RNA aptamer (RNA probe; reporter region) simulated in the third aspect of the fourth embodiment (FIG. 15A). Here, 3WJdB, a modified form of a fluorescent RNA aptamer called Broccoli, was bonded to each Cas protein-binding RNA to prepare a complexed RNA molecule. The 3WJdB emits fluorescence with DFHBI-1T as a substrate. Hence, when the target substance (Cas protein) is absent in the reaction system, no fluorescence is emitted because the complexed RNA molecule is degraded by XRN1. On the other hand, in the presence of the target substance, fluorescence is generated by the action of 3WJdB and DFHBI-1T because the degradation of the complexed RNA molecule is inhibited (FIG. 15B). In order to confirm whether such a phenomenon occurs and changes in degradation, change in fluorescence was observed after addition of the target substance and XRN1.

FIG. 15C shows a condition of the presence or absence (+/-) of a substance and an enzyme contained in each sample reaction system, and the type of the complexed RNA molecule. Immediately after addition of the substance and the enzyme, high fluorescence is observed because the degradation of the RNA molecule does not yet progress. By contrast, the fluorescence is reduced over time because the degradation progresses. FIGs. 15D and 15E show change in fluorescence under conditions of SaCas9-binding RNA and SpCas9-binding RNA, respectively. The samples 8 and 16 of FIG. 15C employed an RNA molecule containing no 3WJdB (which does not correspond to the complexed RNA molecule) and thus exhibited change in fluorescence of DFHBI-1T itself. The samples 1 and 9 were not supplemented with DFHBI-1T and generated no fluorescence. The samples 2 to 4 and 10 to 12 were not supplemented with XRN1, and RNA degradation did not progress. As shown therein, the attenuation of fluorescence was suppressed in the presence of the corresponding Cas9 protein under the condition supplemented with XRN1 (samples 6 and 15). On the other hand, the attenuation of fluorescence was shown in the absence of the corresponding Cas9 protein (samples 5, 7, 13, and 14). These results suggested that the presence or absence of a target substance in a reaction solution can be determined using the present ON switch mechanism on the basis of this difference in the amount of fluorescence.

## Claims

1. An RNA molecule comprising:
(a) a recognition region that specifically recognizes a target substance;
(b) a cap-independent translational activation region positioned on the 3' terminal side of the recognition region; and
(c) a coding region positioned on the 3' terminal side of the translational activation region, the coding region being translated by the translational activation region, wherein
the RNA molecule does not contain 5' cap structure.

2. The RNA molecule according to claim 1, wherein the recognition region comprises a nucleic acid sequence selected from an RNA aptamer, aptazyme, and a nucleic acid sequence that specifically recognizes RNA.

3. The RNA molecule according to claim 1, wherein the translational activation region is selected from an internal ribosome entry site (IRES), a ribosome binding site, and a translational activator recognition site.

4. The RNA molecule according to claim 1, wherein the coding region comprises a nucleic acid sequence encoding a detectable protein, a therapeutic protein, or an RNA-binding protein.

5. The RNA molecule according to claim 1, further comprising a spacer region linked to the 5' side of the recognition region.

6. The RNA molecule according to claim 1, wherein
the recognition region is linked to the 5' side of the translational activation region, and
the translational activation region is linked to the 5' side of the coding region.

7. An RNA molecule comprising:
(A) a recognition region that specifically recognizes a target substance; and
(B) a reporter region positioned on the 3' terminal side of the recognition region, wherein
the RNA molecule does not contain a 5' cap structure.

8. The RNA molecule according to claim 7, wherein the recognition region comprises a nucleic acid sequence selected from an RNA aptamer, aptazyme, and a nucleic acid sequence that specifically recognizes RNA.

9. The RNA molecule according to claim 7, wherein the reporter region comprises a light-up aptamer that generates an optical signal by binding to an RNA probe.

10. A DNA construct comprising:
a DNA sequence encoding an RNA molecule according to claim 1 or 7; and
a 5' cap structure-removing sequence.

11. The DNA construct according to claim 10, wherein the 5' cap structure-removing sequence encodes a self-cleaving ribozyme, a miRNA target sequence, or an RNA-degrading sequence.

12. A method for producing the RNA molecule according to claim 1 or 7, comprising steps of:
obtaining an RNA molecule comprising: a 5' cap structure; a recognition region that specifically recognizes a target substance; a cap-independent translational activation region positioned on the 3' terminal side of the recognition region; and a coding region positioned on the 3' terminal side of the translational activation region, the coding region being translated by the translational activation region; and
removing the 5' cap structure from the RNA molecule.

13. A method for detecting a target substance, comprising bringing the RNA molecule according to claim 1 or 7 into contact with a cell.

14. A method for detecting a target substance, comprising introducing the DNA construct according to claim 10 into a cell.

15. A method for detecting a target substance, comprising adding exoribonuclease and the RNA molecule according to claim 1 or a DNA construct comprising a DNA sequence encoding the RNA molecule and a 5' cap structure-removing sequence to a cell-free gene expression system.

16. A method for detecting a target substance, comprising steps of:
adding exoribonuclease, the RNA molecule according to claim 7 or a DNA construct comprising a DNA sequence encoding the RNA molecule and a 5' cap structure-removing sequence, and an RNA probe that generates an optical signal by binding to the reporter region of the RNA molecule, to a sample; and
detecting the optical signal.

17. A translational regulation system comprising the RNA molecule according to claim 1 or a DNA construct comprising a DNA sequence encoding the RNA molecule and a 5' cap structure-removing sequence.

18. A translational regulation system comprising at least two RNA molecule groups,
wherein each of the RNA molecule groups comprises a plurality of RNA molecules each comprising: a recognition region that specifically recognizes a target substance; a cap-independent translational activation region positioned on the 3' terminal side of the recognition region; and a coding region positioned on the 3' terminal side of the translational activation region, the coding region being translated by the translational activation region, wherein each of the RNA molecules does not contain a 5' cap structure, and
wherein the target substance that is specifically recognized by the recognition region differs for each of the RNA molecule groups.
